# EUROPÄISCHE PATENTSCHRIFT

(11) **EP 4 118 431 B1**
(45) Veröffentlichungstag und Bekanntmachung des Hinweises auf die Patenterteilung: **06.05.2026**
(21) Anmeldenummer: 21711847.0
(22) Anmeldetag: 11.03.2021
(51) Int. Cl.: G01N 33/50, G01N 33/575, A61K 31/436, G01N 33/57515, A61K 49/00, A61P 35/00, A61K 31/713, A61K 45/06, A61K 47/55

(54) **INHIBITION VON FKBP1A ZUR THERAPIE DES TRIPLE-NEGATIVEN MAMMAKARZINOMS**
INHIBITION OF FKBP1A FOR THE TREATMENT OF TRIPLE-NEGATIVE MAMMARY CARCINOMA
INHIBITION DE FKBP1A POUR LA THÉRAPIE DU CARCINOME MAMMAIRE TRIPLE NÉGATIF

(30) Priorität: 12.03.2020 DE 102020203224; 25.06.2020 DE 102020207900
(43) Veröffentlichungstag der Anmeldung: 18.01.2023
(73) Patentinhaber: Heinrich-Heine-Universität Düsseldorf, 40225 Düsseldorf (DE)
(72) Erfinder: ESSER, Knud, 51061 Köln (DE); KULIK, Andrea, 81475 München (DE); FEHM, Tanja, 40225 Düsseldorf (DE); STEIMEL, Judith, 45219 Essen (DE)
(74) Vertreter: Kutzenberger Wolff & Partner
(86) Internationale Anmeldenummer: PCT/EP2021/056164
(87) Internationale Veröffentlichungsnummer: WO 2021/180840

(56) Entgegenhaltungen:
- WO-A1-2014/006115
- WO-A1-2015/169951
- WO-A1-2018/185341
- US-A1- 2009 215 812
- JASMEET CHADHA SINGH ET AL: "Phase 2 trial of everolimus and carboplatin combination in patients with triple negative metastatic breast cancer", BREAST CANCER RESEARCH, CURRENT MEDICINE GROUP LTD, GB, vol. 16, no. 2, 31 March 2014 (2014-03-31), pages R32, XP021182620, ISSN: 1465-5411, DOI: 10.1186/BCR3634
- BASHO REVA K. ET AL: "Comparative Effectiveness of an mTOR-Based Systemic Therapy Regimen in Advanced, Metaplastic and Nonmetaplastic Triple-Negative Breast Cancer", THE ONCOLOGIST, vol. 23, no. 11, 23 August 2018 (2018-08-23), pages 1300 - 1309, XP093317694, ISSN: 1083-7159, DOI: 10.1634/theoncologist.2017-0498
- LEE JIN SUN ET AL: "Phase I clinical trial of the combination of eribulin and everolimus in patients with metastatic triple-negative breast cancer", BREAST CANCER RESEARCH (ONLINE EDITION), vol. 21, no. 1, 8 November 2019 (2019-11-08), United Kingdom, Netherlands, United States, XP093317688, ISSN: 1465-542X, DOI: 10.1186/s13058-019-1202-4
- HE JICHAO ET AL: "Multi-targeted kinase inhibition alleviates mTOR inhibitor resistance in triple-negative breast cancer", BREAST CANCER RESEARCH AND TREATMENT, SPRINGER , NY, US, vol. 178, no. 2, 6 August 2019 (2019-08-06), pages 263 - 274, XP036908599, ISSN: 0167-6806, [retrieved on 20190806], DOI: 10.1007/S10549-019-05380-Z
- COSTA RICARDO L ET AL: "Targeting the PI3K/AKT/mTOR pathway in triple-negative breast cancer: a review", BREAST CANCER RESEARCH AND TREATMENT, SPRINGER , NY, US, vol. 169, no. 3, 7 February 2018 (2018-02-07), pages 397 - 406, XP036504009, ISSN: 0167-6806, [retrieved on 20180207], DOI: 10.1007/S10549-018-4697-Y
- DE PRADIP ET AL: "Promise of rapalogues versus mTOR kinase inhibitors in subset specific breast cancer: Old targets new hope", CANCER TREATMENT REVIEWS, ELSEVIER, AMSTERDAM, NL, vol. 39, no. 5, 23 January 2013 (2013-01-23), pages 403 - 412, XP028593072, ISSN: 0305-7372, DOI: 10.1016/J.CTRV.2012.12.002
- YARDLEY: "Combining mTOR Inhibitors with Chemotherapy and Other Targeted Therapies in Advanced Breast Cancer: Rationale, Clinical Experience, and Future Directions", BREAST CANCER: BASIC AND CLINICAL RESEARCH, 1 February 2013 (2013-02-01), pages 7, XP055409544, DOI: 10.4137/BCBCR.S10071
- JÜRGEN M. KOLOS ET AL: "FKBP Ligands-Where We Are and Where to Go?", FRONTIERS IN PHARMACOLOGY, vol. 9, 5 December 2018 (2018-12-05), XP055704150, DOI: 10.3389/fphar.2018.01425
- XING MINGYOU ET AL: "FKBP12 is a predictive biomarker for efficacy of anthracycline-based chemotherapy in breast cancer", CANCER CHEMOTHERAPY AND PHARMACOLOGY, SPRINGER VERLAG , BERLIN, DE, vol. 84, no. 4, 19 August 2019 (2019-08-19), pages 861 - 872, XP036895441, ISSN: 0344-5704, [retrieved on 20190819], DOI: 10.1007/S00280-019-03923-1

## Beschreibung

Es werden die Prioritäten beansprucht von deutscher Patentanmeldung Nr. 10 2020 203 224.6, eingereicht am 12. März 2020, sowie von deutscher Patentanmeldung Nr. 10 2020 207 900.5, eingereicht am 25. Juni 2020.

Die Erfindung betrifft ein Verfahren zum Auffinden von Inhibitoren der Peptidyl-prolyl cis-trans Isomerase FKBP1A oder von Antikörpern, Proteinen oder Molekülen mit spezifischer Affinität zu FKBP1A. Die Erfindung betrifft außerdem FKBP1A-spezifische siRNA, Inhibitoren der Expression von FKBP1A, Inhibitoren der enzymatischen Aktivität von FKBP1A, sowie Inhibitoren der Interaktion(en) von FKBP1A mit Interaktionspartner(n) jeweils zur Behandlung von Erkrankungen, insbesondere Krebserkrankungen oder neurodegenerative Erkrankungen. Darüber hinaus betrifft die Erfindung die Verwendung von FKBP1A als prognostischer oder diagnostischer Marker von Krebserkrankungen. Bei den Krebserkrankungen handelt es sich bevorzugt um das Mammakarzinom, insbesondere um das Triple-negative Mammakarzinom (TNBC, *triple-negative breast cancer*), ganz bevorzugt um den mesenchymal stem-like Subtyp. Die unabhängigen Ansprüche 1, 13 und 14 definieren die Erfindung. Die abhängigen Ansprüche definieren bevorzugte Ausführungsformen der Erfindung.

Das Triple-negative Mammakarzinom wird bei 10-15% aller Brustkrebspatientinnen diagnostiziert und ist durch eine hohe Rezidivrate, aggressives Wachstum, frühe Metastasierung und schlechte Prognose charakterisiert. Kennzeichnend und wahrscheinlich mitursächlich für diese hohe Aggressivität sind der epigenetisch fehlgesteuerte geringe Differenzierungsstatus sowie der hohe Stammzellcharakter der Tumorzellen. Dies trifft insbesondere auf den mesenchymal stem-like Subtyp des TNBC zu.

Im Gegensatz zu anderen Subformen des Mammakarzinoms wie z.B. des Hormonrezeptor- oder des Her2-positiven Mammakarzinoms besteht die aktuelle Therapie des Triple-negativen Mammakarzinoms gemäß den aktuellen Leitlinien ausschließlich in einer Behandlung mit Chemotherapeutika. Diese wirken jedoch unspezifisch mit bedeutenden Nebenwirkungen im gesunden Gewebe. Bis heute stehen beim Triple-negativen Mammakarzinom aufgrund der negativen Expression bekannter onkologischer Zielstrukturen (Östrogenrezeptor, Progesteronrezeptor oder Her2-Rezeptor) keine pharmakologisch adressierbaren zellulären Zielstrukturen für Therapieansätze zur Verfügung (vgl. Kumar, P. & Aggarwal, R. An overview of triple-negative breast cancer. Archives of Gynecology and Obstetrics (2016). doi:10.1007/s00404-015-3859-y).

Viele zielgerichtete Therapieansätzen wurden bzw. werden in klinischen Studien untersucht, wie z.B. der Einsatz von PARP-Inhibitoren, Angiogenese-Inhibitoren oder mTOR-Inhibitoren, oder eine EGFR-gerichtete Behandlung. Diese Ansätze zeigten jedoch nur eine begrenzte therapeutische Effizienz (Fedele, P., Orlando, L. & Cinieri, S. Targeting triple negative breast cancer with histone deacetylase inhibitors. Expert Opinion on Investigational Drugs (2017). doi:10.1080/13543784.2017.1386172). Bis heute konnte keine effiziente personalisierte Therapie des Triple-negativen Mammakarzinoms entwickelt werden.

Epigenetische Wirkstoffe, z.B. HDAC-Inhibitoren, befinden sich ebenfalls in ersten klinischen Studien zur Behandlung des Triple-negativen Mammakarzinoms. Dem Ansatz, mit epigenetischen Wirkstoffen den hohen Stammzellcharakter, die geringe Apoptoseinduktion sowie die geringe Immunogenität der Krebszellen zu adressieren, wird ein großes therapeutisches Potential zugesprochen (Fedele, P., Orlando, L. & Cinieri, S. Targeting triple negative breast cancer with histone deacetylase inhibitors. Expert Opinion on Investigational Drugs (2017). doi:10.1080/13543784.2017.1386172; Mazzone, R., Zwergel, C., Mai, A. & Valente, S. Epi-drugs in combination with immunotherapy: a new avenue to improve anticancer efficacy. Clinical Epigenetics (2017). doi:10.1186/s13148-017-0358-y). Die aktuell untersuchten Wirkstoffe zielen jedoch lediglich auf allgemeine zelluläre epigenetische Prozesse und sind wenig tumorspezifisch (Roberti, A., Valdes, A. F., Torrecillas, R., Fraga, M. F. & Fernandez, A. F. Epigenetics in cancer therapy and nanomedicine. Clinical Epigenetics (2019). doi:10.1186/s13148-019-0675-4).

US 2009/0215812 betrifft Zusammensetzungen und Verfahren zum Bewerten der Wahrscheinlichkeit, dass ein Tumor gegenüber einem mTOR-Inhibitor, z. B. Rapamycin oder einem Rapamycin-Analogon, empfindlich ist.

US 2016/0235731 betrifft bifunktionelle Verbindungen, die als Proteinabbau induzierende Einheiten wirken und Verfahren zum gezielten Abbau endogener Proteine durch Verwendung der bifunktionellen Verbindungen, die eine Cereblon-bindende Einheit an einen Liganden binden, der in der Lage ist, an das Zielprotein zu binden, das bei der Behandlung von proliferativen Störungen verwendet werden kann.

US 2019/0092788 betrifft 32-Desoxo-Rapamycin-Derivate und deren Verwendungsverfahren.

WO 2009/030770 betrifft Verfahren und Werkzeuge zum Erhalten einer effizienten Prognose (Prognose) von Brustkrebs-Östrogenrezeptor (ER)-negativen Patientinnen, wobei die Immunantwort der Schlüsselakteur der Brustkrebs-Prognose ist.

WO 2013/093493 betrifft ein neues Rapamycin-Analogon, Verfahren zu seiner Herstellung und seiner Verwendung in der Therapie, insbesondere zur Behandlung von Lupus und / oder Multipler Sklerose (MS).

S. Siamakpour-Reihani, PLoS ONE, 2011, Vol. 6, Art. e20412 betrifft das immunsuppressive Medikament Tacrolimus (FK506), welches in der Lage ist, die durch SFRP2 und VEGF stimulierte Invitro-Tubusbildung zu hemmen und die Migration von Endothel- und Brustkrebszellen zu hemmen. Tacrolimus könnte bei der Behandlung von Brustkrebs besonders nützlich sein, da es das Wachstum von Brusttumor-Xenograft in vivo abschwächt und FKBP12 als Adaptermolekül zu Calcineurin in der Vaskulatur menschlicher Brustkarzinome exprimiert wird.
Des Weiteren kann auf folgende Veröffentlichungen verwiesen werden:
- WO 2018/185341 A1 und WO 2014/006115 A1;
- HE JICHAO ET AL: "Multi-targeted kinase inhibition alleviates mTOR inhibitor resistance in triple-negative breast cancer", BREAST CANCER RESEARCH AND TREATMENT, SPRINGER, NY, US, Bd. 178, Nr. 2, 6. August 2019, Seiten 263-274;
- COSTA RICARDO L ET AL: "Targeting the PI3K/AKT/mTOR pathway in triple-negative breast cancer: a review", BREAST CANCER RESEARCH AND TREATMENT, SPRINGER, NY, US, Bd. 169, Nr. 3, 7. Februar 2018, Seiten 397-406:
- JASMEET CHADHA SINGH ET AL: "Phase 2 trial of everolimus and carboplatin combination in patients with triple negative metastatic breast cancer", BREAST CANCER RESEARCH, CURRENT MEDICINE GROUP LTD, GB, Bd. 16, Nr. 2, 31. März 2014 (2014-03-31), Seite R32;
- Basho Reva K. ET AL: "Comparative Effectiveness of an mTOR-Based Systemic Therapy Regimen in Advanced, Metaplastic and Nonmetaplastic Triple-Negative Breast Cancer", THE ONCOLO-GIST, Bd. 23, Nr. 11, 23. August 2018 (2018-08-23), Seiten 1300-1309; und
- Lee Jin Sun ET AL: "Phase I clinical trial of the combination of eribulin and everolimus in patients with metastatic triple-negative breast cancer", Breast Cancer Research (Online Edition), Bd. 21, Nr. 1, 8. November 2019.

Das Bestreben der zukünftigen personalisierten Krebstherapie ist eine nebenwirkungsarme und auf den Tumor gerichtete Behandlung, welche speziell pathophysiologische Veränderungen der Krebszellen adressiert. Es besteht beim Triple-negativen Mammakarzinom ein großer Bedarf an neuartigen, zielgerichteten Therapieansätzen, welche nicht das gesunde Gewebe, sondern lediglich spezifisch die Tumorzellen angreifen.

Es ist eine Aufgabe der Erfindung, neue Ansätze zur Therapie von Krebserkrankungen bereitzustellen, insbesondere zur Therapie des Triple-negativen Mammakarzinoms, welche Vorteile gegenüber dem Stand der Technik haben. Die Therapieansätze sollten zielgerichtet sein, d.h. lediglich die Tumorzellen angreifen, nicht jedoch das gesunde Gewebe. Darüber hinaus ist es eine Aufgabe der Erfindung, neue Diagnostizieransätze für Krebserkrankungen, insbesondere für das Triple-negative Mammakarzinom bereitzustellen, welche Vorteile gegenüber dem Stand der Technik haben.

Diese Aufgaben werden durch den Gegenstand der Patentansprüche gelöst.

Es wurde überraschend gefunden, dass die Peptidyl-prolyl cis-trans Isomerase *"FK506 binding protein 1a"* (FKBP1A) als tumorspezifische Zielstruktur im Triple-negativen Mammakarzinom geeignet ist. FKBP1A eröffnet dabei vielfältige Möglichkeiten für neue zielgerichtete Therapieansätze und auch für neue Diagnostizieransätze.

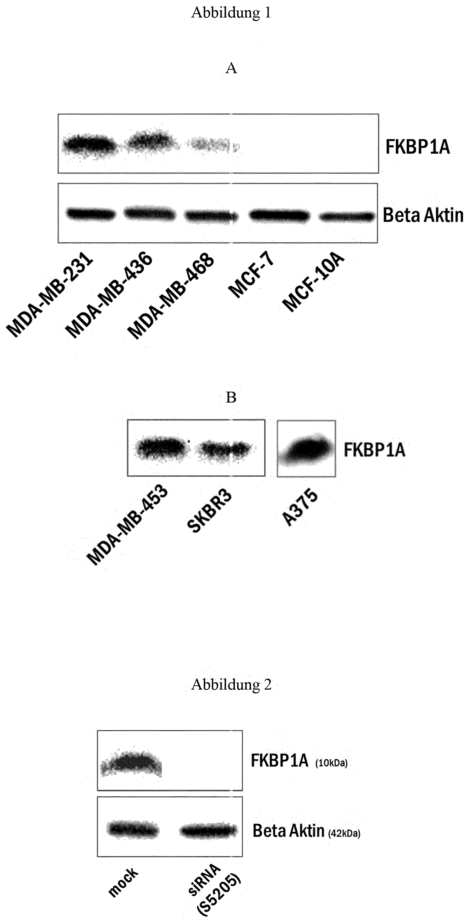

In verschiedenen Tumorzellmodellen konnte gezeigt werden, dass FKBP1A in Triple-negativen Brustkrebszellen, insbesondere dem mesenchymal stem-like Subtyp, in hohen Maße exprimiert wird, im Unterschied dazu in Hormonrezeptor-positiven Brustkrebszellen oder in nicht-malignen Zellen jedoch nicht nachweisbar ist (vgl. Abbildung 1A). Neben Tumormodellen des Triple-negativen Mammakarzinoms konnte die hohe Expression von FKBP1A auch in Zellen des Her2-positiven, Hormonrezeptor-negativen Mammakarzinoms sowie des malignen Melanoms gezeigt werden (vgl. Abbildung 1B).

Es wurde überraschend gefunden, dass unter den Triple-negativen Brustkrebszellen solche in besonders hohem Maße FKBP1A exprimieren, welche vom mesenchymalen stem-like Subtyp sind, d.h. einen hohen Stammzellcharakter haben, wie z.B. MDA-MB-231 und MDA-MB-436, welche kommerziell verfügbar sind. Damit sind Brustkrebszellen des mesenchymalen stem-like Subtyps besonders gut für das erfindungsgemäße Verfahren geeignet, da sie eine gute Diskriminierung zwischen wirksamen und nicht wirksamen Inhibitoren ermöglichen.

Zum heutigen wissenschaftlichen Stand ist lediglich die negative Proteinexpression von FKBP1A in luminalen MCF-7-Zellen oder in gesundem Brustgewebe bekannt (vgl. https://www.prote-inatlas.org/ENSG00000088832-FKBP1A/pathology; https://www.proteinat-las.org/ENSG00000088832-FKBP1A/tissue).

Ferner wurde überraschend gefunden, dass FKBP1A eine wesentliche Rolle dabei spielt, die bis heute in der Tumorpathogenese wenig verstandene Inhibierung der physiologischen Differenzierung der Triple-negativen Brustkrebszellen zu bewirken und die Krebszellen in einem gering differenzierten Zustand zu arretieren. Sollte sich in ergänzenden Untersuchungen herausstellen, dass FKBP1A auch in Zellen der Tumormikroumgebung, wie z.B. in Tumor-assoziierten Fibroblasten, Makrophagen oder besonders Stammzellen, insbesondere mesenchymalen Stammzellen, exprimiert wird, würde ein auf diesem Therapieansatz basierendes neuartiges Therapeutikum somit eine erhöhte Expression nicht nur in Tumorzellen, sondern auch in Zellen der Tumormikroumgebung adressieren und dadurch die Anti-Tumorwirkung weiter verstärken (He, W. et al. MSC-regulated lncRNA MACC1-AS1 promotes stemness and chemoresistance through fatty acid oxidation in gastric cancer, Oncogene (2019), doi:10.1038/s41388-019-0747-0). Darüber hinaus wird von den Erfindern aufgrund des charakteristischen hohen Stammzellcharakters der Krebszellen des Triple-negativen Brustkrebses, hier insbesondere des mesenchymal stem-like Subtyps, eine vergleichbare zu der hier beschriebenen Rolle von FKBP1A in sogenannten Tumorstammzellen anderer Krebserkrankungen erwartet. Insbesondere Krebszellen des mesenchymal stem-like Subtyps, wie z.B. MDA-MB-231, besitzen einen besonders hohen Anteil an Tumorstammzellen. Tumorstammzellen sind eine Subpopulation von Krebszellen, welcher in der Tumorpathophysiologie von Krebserkrankungen eine wesentliche Rolle hinsichtlich Therapie-Resistenz und Metastasierung zugesprochen wird (Ku 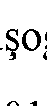 oǧlu, A. & Biray Avci, Ç. Cancer stem cells: A brief review of the current status, Gene (2019), doi:10.1016/j.gene.2018.09.052). Eine FKBP1A-zielgerichtete Therapie ist neuartig somit nicht nur spezifisch gegen Krebszellen des Triple-negativen Mammakarzinoms und hier speziell der Tumorstammzellen, sondern auch gegen die Subpopulation von Tumorstammzellen anderer Krebserkrankungen spezifisch.

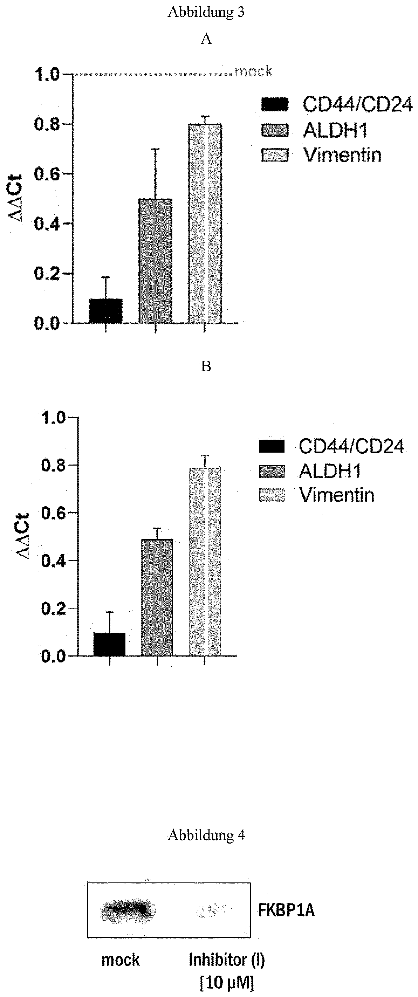

Darüber hinaus wurde überraschend gefunden, dass über eine gerichtete Expressionsreduktion von FKBP1A mittels spezifischer siRNA (siehe Abbildung 2) FKBP1A therapeutisch als tumorspezifisches Target beim Triple-negativen Brustkrebs, insbesondere des mesenchymal stem-like Subtyps, charakterisiert und adressiert werden kann. Dies ist u.a. auch für die aktuelle Immuntherapie von Bedeutung (Bu, X., Yao, Y. & Li, X. Immune checkpoint blockade in breast cancer therapy. in Advances in Experimental Medicine and Biology (2017). doi:10.1007/978-981-10-6020-5_18). So kann mit Hilfe des erfindungsgemäßen Therapieansatzes die aktuelle Therapie durch
- gerichtete Reduktion des zellulären (Tumor-)Stammzellcharakters der Krebszellen,
- Reduktion des mesenchymalen Charakters der Krebszellen,
- Erhöhung des zellulären Differenzierungsstatus',
- Verstärkung der zellulären Apoptoseinduktion sowie
- Erhöhung der zellulären Immunogenität,
insbesondere in einer synergistischen Behandlung zusammen mit der Immuntherapie, adjuvant wesentlich verbessert werden (siehe Abbildung 3).

Erfindungsgemäß wird über Verwendung von siRNAs, Antikörpern oder Pharmaka, welche jeweils für FKBP1A spezifisch bzw. selektiv sind, eine personalisierte Medizin bei der Behandlung des Triple-negativen Mammakarzinoms ermöglicht.

Die Tumorzellen des TNBC, insbesondere des mesenchymal stem-like Subtyps, besitzen eine besonders hohe Metastasierungstendenz. Neben dem beschriebenen hohen Tumorstammzellcharakter besitzen diese Tumorzellen eine besonders hohe mesenchymale Zellcharakteristik und sind durch die starke Expression an Markern der Epithelial-Mesenchymal Transition (EMT) charakterisiert. Überraschenderweise wurde gefunden, dass die Herabregulation von FKBP1A zu einer Verringerung von EMT-Markern wie Vimentin führt (s. Abbildung 3A). Darüber hinaus wurde überraschend festgestellt, dass beim Mammakarzinom die Expression von FKBP1A mit der Expression von TGF-beta-Rezeptor II, einem über Bindung an seinen Liganden TGF-beta wesentlichen Induktor von EMT, korreliert. Aus diesem Grund wird eine starke Expression von FKBP1A in einzelnen oder in Gruppen die den Tumor umgebende Extrazellulär-Matrix invasierenden Tumorzellen erwartet. Eine FKBP1A-zielgerichtete Therapie ist neuartig somit nicht nur spezifisch gegen Krebszellen des Triple-negativen Mammakarzinoms, sondern auch gegen die Subpopulation von (bei epithelialen Zellen: nach einer EMT) die Tumor-Extrazellulär-Matrix (auch von ursprünglich FKBP1A negativem oder gering exprimierendem Tumorgewebe) invasierenden Krebszellen spezifisch.

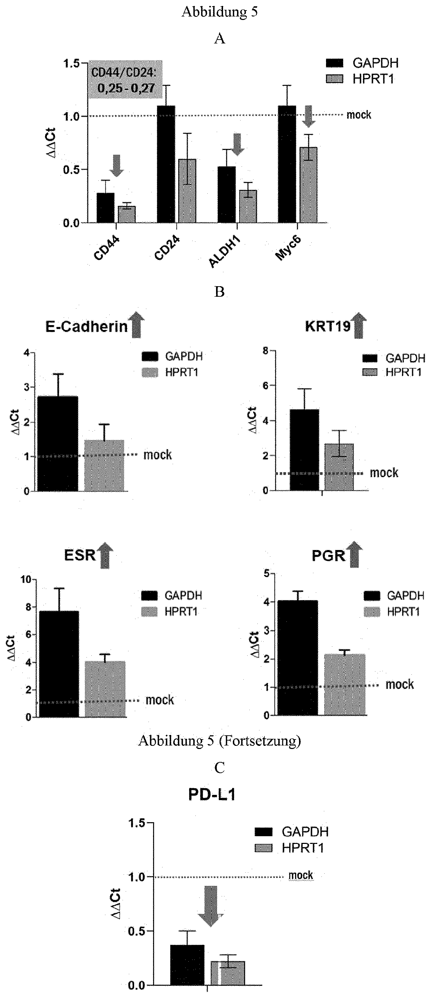

Für erste Wirkstoffkandidaten konnte experimentell gezeigt werden, dass sie beim Triple-negativen Mammakarzinom eine Expressionsreduktion von FKBP1A induzieren (siehe Abbildung 4) und ebenfalls die Expressionsreduktion von (Tumor-)Stammzellmarkern bewirken (siehe Abbildung 5). Darüber hinaus konnte gefunden werden, dass sie eine metabolische Reprogrammierung induzieren. Für bestimmte Makrolide (z.B. Rapamycin bzw. Rapamycin-Derivate wie z.B. Everolismus (RAD001), Temsirolimus (CCI-779) oder Ridaforolimus (AP23573)), von denen einzelne für die antibiotische Therapie beim Menschen zugelassen sind, oder für FK506 ist eine inhibitorische Wirkung auf FKBP1A beschrieben. Diese Inhibitoren adressieren über FKBP1A als Adaptermolekül mTOR bzw. Calcineurin. Darüber hinaus sind bis heute im Kontext neurologischer Therapieansätze spezifische Inhibitoren von FKBP1A entwickelt worden, die im Gegensatz zu Rapamycin oder FK506 nicht zusätzlich an mTOR bzw. Calcineurin binden, sondern FKBP1A isoliert adressieren und somit keine immunsuppressive Wirkung aufweisen. Dies ist insbesondere im Rahmen hier angestrebter synergistischer Immuntherapien des Triple-negativen Mamma-Karzinoms von großer Bedeutung. Darüber hinaus bezieht sich die beschriebene Anti-Tumorwirkung auf eine isolierte Adressierung von FKBP1A, da ohne die genannten mTOR- bzw. Calcineurin-spezifischen Inhibitoren eine Interaktion von FKBP1A mit diesen Proteinen nicht beschrieben ist. Zu diesen isoliert die FKBP1A-Aktivität adressierenden Inhibitoren gehören u.a. ElteN378, V-10367 (Vertex, USA), GPI-1046 (Guilford, USA), GPI-1485 (Guilford, USA), SLF, FK1706, FK-1012, AG5437 oder AG5507 (Kolos, J. M., Voll, A. M., Bauder, M. & Hausch, F. FKBP Ligands - Where We Are and Where to Go? Front. Pharmacol. (2018), doi:10.3389/fphar.2018.01425). Die Verwendung solcher und weiterer Inhibitoren zur Krebstherapie, insbesondere zur Therapie des Triple-negativen Mammakarzinoms im epigenetischen Kontext, könnte eine wesentliche Verbesserung in der gezielten Krebstherapie darstellen.

Es wurde entgegen US 2009/0215812 überraschend gefunden, dass mTOR-Inhibitoren bei der Behandlung des TNBC keine wesentliche Wirkung zeigen. Auch in Zellkultur zeigen mTOR-Inhibitoren in Modellen des TNBC, insbesondere des mesenchymal stem-like Subtyps keine Wirkung, wie z.B. in MDA-MB-231-Zellen. Experimentelle Erkenntnisse zeigen sogar eine Verminderung der ApoptoseInduktion nach Inkubation von MDA-MB-231-Zellen mit Rapamycin-Derivaten. Da FKBP1A, insbesondere im mesenchymal-stem-like Subtyp des TNBC, wie z.B. den MDA-MB-231-Zellen, stark exprimiert ist, kann die in US 2009/0215812 postulierte Korrelation von FKBP1A-Expression und Anti-Tumorwirkung durch Rapamycin-Derivate insbesondere für den Mesenchymal-Subtyp nicht bestätigt werden. In US 2009/0215812 wird eine verstärkte Anti-Tumoraktivität durch FKBP1A beschrieben, wenn FKBP1A verstärkt in den Tumorzellen exprimiert wird, also gerade das Gegenteil der erfindungsgemäßen Korrelation. Darüber hinaus wird in US 2009/0215812 keine pro-cancerogene Wirkung in Korrelation mit der alleinigen Expression oder Aktivität von FKBP1A insbesondere in Hinsicht auf die Tumorstammzellcharakteristik und/oder Induktoren der EMT dargestellt, insbesondere nicht beim TNBC und ganz besonders nicht beim mesenchymal stem-like Subtyp.

Zudem wurde entgegen Siamakpour-Reihani et al. überraschend gefunden, dass eine pro-cancerogene Wirkung in Korrelation mit der alleinigen Expression oder Aktivität von FKBP1A insbesondere in Hinsicht auf die Tumorstammzellcharakteristik und/oder Induktoren der EMT besteht, insbesondere im TNBC und ganz insbesondere im mesenchymal-stem-like Subtyp. Die von Siamakpour-Reihani et al. beschriebene Anti-Tumorwirkung der FKBP1A-bindenden Moleküle (u.a. Tacrolimus) wird ausschließlich über die Bindung an das Zielmolekül Calcineurin beschrieben, in der FKBP1A lediglich als Adaptermolekül für Tacrolimus dient.

In US 2019/0092788 wird die Expression von FKBP1A in Tumorzellen als Indikator für eine erfolgreiche Inhibition des mTOR-Signalweges über Rapamycin-Derivate beschrieben. Es werden Derivate beschrieben, deren mTOR-Inaktivierung unterschiedlich stark auf eine Expression von FKBP1A bezogen wird. Eine Anti-Tumorwirkung durch direkte und alleinige Adressierung von FKBP1A wird nicht beschrieben, insbesondere auch nicht in Hinsicht auf die Tumorstammzellcharakteristik und/oder Induktoren der EMT, insbesondere nicht beim TNBC und ganz besonders nicht beim mesenchymal stem-like Subtyp.

In WO 2013/093493 werden Rapamycin-Derivate beschrieben, die zusätzlich zur Bindung und Inhibition von mTOR eine unterschiedlich starke Inhibition der PPI von FKBP1A aufzeigen. Dabei wird dargelegt, dass Derivate, die eine stärkere PPI-Inhibition von FKBP1A bewirken, eine verstärkte Inhibition des Zellwachstums von SF268, U87MG (beides Glioblastom), DU145 und PC3 (beides Prostata-Karzinom) aufzeigen. Dabei werden sehr hohe Konzentrationen der Inhibitoren im mikromolaren Bereich verwendet, während für Rapamycin-Derivate eine starke Wirkung bereits im sub-nanomolaren Bereich gezeigt ist. Ein Off-Target-Effekt erscheint in diesem Zusammenhang wahrscheinlich. Eine Anti-Tumorwirkung durch direkte und alleinige Adressierung von FKBP1A in Hinsicht auf die Tumorstammzellcharakteristik und/oder Induktoren der EMT wird nicht beschrieben, insbesondere nicht beim TNBC und ganz besonders nicht beim mesenchymal stem-like Subtyp. Es konnte keine wesentliche Wachstumsinhibition durch isolierte Adressierung von insbesondere der enzymatischen Aktivität FKBP1A in den Tumorzellen gezeigt werden. Darüber hinaus deuten experimentelle Erkenntnisse daraufhin, dass die beschriebene Anti-Tumorwirkung im Gegensatz zu WO 2013/093493 unabhängig von der PPI-Aktivität der FKBP1A sein könnte.

In US 2016/0235731 werden Daten zur Reduktion der zellulären Proteinkonzentration von FKBP1A-Knockdown mittels FKBP1A-adressierender PROTCAs in der AML-Zell-Linie MV4-11 cells gezeigt. Eine Anti-Tumorwirkung des Knockdowns wird nicht dargestellt. Es wird lediglich auf das bekannte onkogene Signaling von FKBP1A verwiesen. Dabei ist als bekannt zu setzen, dass FKBP1A die TGF-beta-Rezeptor-I-Aktivität inhibiert. Im Gegensatz dazu wurde überraschend gefunden, dass FKBP1A besonders beim TNBC und ganz besonders im mesenchymal stem-like Subtyp besonders stark exprimiert wird, für welche wiederum bekannt ist, dass TGF-beta über Aktivierung des TGF-Beta-Rezeptoren eine starke Epithelial-mesenchymale-Transition induziert. Eine gezielte Verminderung der FKBP1A-Expression bzw. Konzentration z.B. durch einen wie in US 2016/0235731 beschriebenen FKABP1A-adressierenden PROTAC sollte nach den wissenschaftlich bekannten Daten demnach besonders beim TNBC und ganz besonders im mesenchymal stem-like Subtyp zu einer Verminderung der TGF-beta-Rezeptor-Inaktivierung durch FKBP1A und damit zu einem verstärkten TGF-beta-Signaling mit bei TNBC bekannter pro-onkogener Wirkung führen.

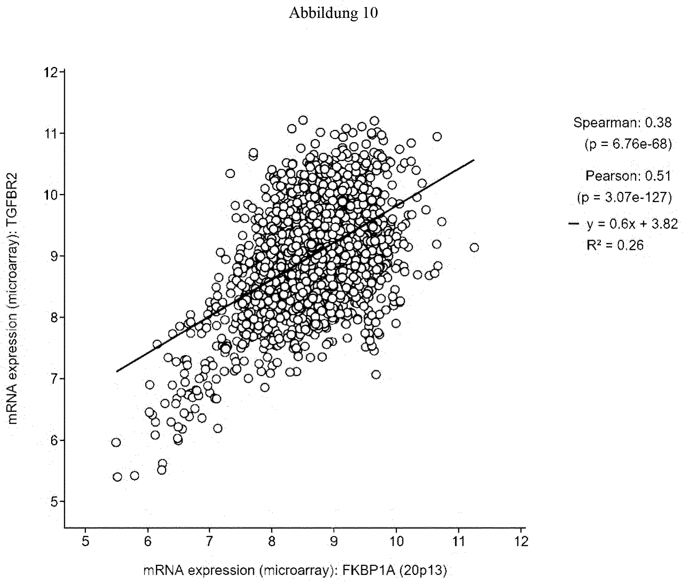

Überraschenderweise induziert die Degradierung von FKBP1A aber eine Reduktion des mesenchymalen Zellcharakters und eine MET. Dieses ist in der Literatur insbesondere für den Triple-negativen Brustkrebs bisher nicht beschrieben. Überraschenderweise konnte in Brustkrebszellen erstmals eine starke Korrelation der Expression von FKBP1A mit dem TGF-beta Rezeptor II dargestellt werden (siehe Abbildung 10). Für TGF-beta Rezeptor I ließ sich diese Korrelation hingegen nicht darstellen. TGF-beta aktivierter TGF-beta Rezeptor II transphosphoryliert den TGF-beta Rezeptor I und induziert EMT, insbesondere im TNBC, hier insbesondere dem "mesenchymal stem like" Subtyp. Diese Daten legen zum ersten Mal nahe, dass FKBP1A über die hohe Expression von TGF-beta Rezeptor II und möglicherweise auch Interaktionen über Aktivierung des TGF-beta Rezeptor I eine Induktion der EMT vermittelt. Diese Erkenntnisse sind bis heute nicht beschrieben und stellen somit einen neuartigen Zusammenhang der starken Expression von FKBP1A dar, insbesondere beim TNBC und insbesondere dem Subtyp "mesenchymal stem like".

Die in WO 2009/030770 für ER- und HER2-negative Brusttumore beschriebene Korrelation einer Krankheitsprognose mit der Expression von FKBP1A innerhalb des definierten Immune-Modules wird als positive Korrelation dargestellt. Demnach ist eine erhöhte Expression von FKBP1A mit einer besseren Prognose für die Patientinnen und geringeren Wahrscheinlichkeit einer Metastasierung verbunden und isoliert auf eine bessere Immunogenizität der Krebszellen und der dadurch resultierenden besser durch das Immunsystem verhinderten Ausbreitung des Tumors und deshalb geringeren Metastasierung bezogen. Vergleichbar wird eine Korrelation der Expression von FKBP1A mit einer besseren Prognose für die Patientinnen und verbundenen geringeren Wahrscheinlichkeit einer Metastasierung auch für HER2+ Tumore beschrieben. Da beide Tumorarten, insbesondere das TNBC, durch eine hohe Malignität charakterisiert sind, wird aus WO 2009/030770 nicht deutlich, warum FKBP1A, hier verbunden mit einer besseren Prognose, zur genauen Typisierung dieser Brustkrebssubtypen herangezogen werden kann. Im Gegensatz hierzu wurde überraschend gefunden, dass eine erhöhte Expression von FKBP1A insbesondere im TNBC, ganz besonders im mesenchymal stem-like-Stubtyp, mit einer erhöhten Expression an Stammzell- und EMT-Marker verbunden ist, woraus im Gegensatz zu WO 2009/030770 eine erhöhte Invasion der Krebszellen, Metastasierung und damit schlechtere Prognose resultiert. Die vorliegende Erfindung zeigt erstmals eine Verwendung als prognostisch ungünstiger Marker insbesondere beim TNBC auf.

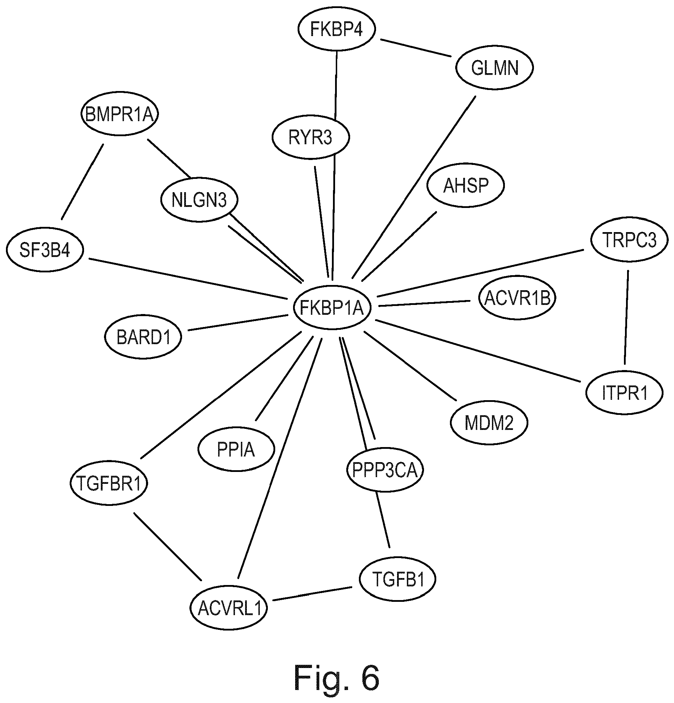

Eine weitere Form der zukünftigen zielgerichteten Therapie ist die Entwicklung sogenannter PROTAC (PROteolysis-TArgeting Chimeras) (An, S. & Fu, L. Small-molecule PROTACs: An emerging and promising approach for the development of targeted therapy drugs, EBioMedicine (2018), doi: 10.1016/j.ebiom.2018.09.005). Hierbei wird das Zielmolekül nicht inhibiert, sondern über spezifische Moleküle mittels gezielter zellulärer Degradierung abgebaut. FKBPs, insbesondere FKBP1A, sind hier häufig verwendete Fusionspartner. Dieser Ansatz kann auch direkt genutzt werden, um zur Behandlung des Triple-negativen Mammakarzinoms die Inaktivierung von FKBPs, bevorzugt FKBP1A, mittels Degradierung zu erreichen. Ein Beispiel für einen solchen Wirkstoff ist FKBP12 PROTAC RC32 (CAS No.: 2375555-66-9). Als Vorstufe kann SLF (CAS No.: 195513-96-3) für die Synthese eines FKBPspezifischen PROTACs verwendet werden. Ein Vorteil der gezielten mittels PROTAC induzierten Proteindegradierung gegenüber einer Inhibition der Enzymfunktion ist, dass über den über PROTACs vermittelten proteolytischen Verdau einer enzymatischen Zielstruktur wie FKBP1A in der Zelle nicht nur dessen enzymatische Aktivität, sondern auch jegliche Protein-Interaktionen unterbunden werden (vgl. [0030] und Abbildung 6). So konnte erstmalig eine stärkere Anti-Tumorwirkung, u.a. auf die genannten Tumorstammzell- und EMT-Marker, durch FKBP12 PROTAC RC32 vermittelte Expressionsreduktion gegenüber reiner Inhibition der Enzymfunktion von FKBP1A festgestellt werden. Eine stärkere Anti-Tumorwirkung (u.a. auf die Zellproliferation) durch Expressionsreduktion gegenüber reiner Inhibition der Enzymfunktion von FKBP1A beim TNBC wurde auch bei siRNA-Knock-down-Versuchen gegenüber Enzyminhibitoren erstmalig festgestellt. Darüber hinaus werden durch PROTACs auch potentielle Bindungspartner ggf. mit abgebaut. Das Wirkungsspektrum ist somit insgesamt wesentlich größer und eine Therapie effizienter. Darüber hinaus wird über die Präsentation von proteolytisch abgebauten Antigenen noch die Immunogenität des Tumors erhöht, was in der synergistischen Immuntherapie eine wesentliche Rolle spielt. Die PROTAC-Technologie kann auch über die Synthese eines nicht nur FKBP1A, sondern dual-targeting Wirkstoffes für den simultanen Abbau zweier Zielstrukturen wie z.B. FKBP1A und PIN1 oder Retinoid-Rezeptoren RAR bzw. RXR zur Therapie des Triple-negativen Mammakarzinoms verwendet werden (vgl. [0062]). Das Design und die Synthese eines solchen Wirkstoffes sind dem Experten bekannt.

Neben FKBP1A sind auch weitere Vertreter der FK506-bindenden Proteine potentielle Zielstrukturen beim Triple-negativen Mammakarzinom und Tumorstammzellen mit der hier für FKBP1A beschriebenen Rolle in der Pathophysiologie der Krebszellen und mit Therapieansätzen mittels spezifischer Inhibitoren oder weiterer hier beschriebener gezielten Adressierungen. Hier sind insbesondere FKBP12.6, FKBP13, FKBP25, FKBP51 und FKPB52 zu nennen (Kolos, J. M., Voll, A. M., Bauder, M. & Hausch, F. FKBP Ligands - Where We Are and Where to Go? Front. Pharmacol. (2018)).

Zusätzlich und unabhängig von einer therapeutischen Anwendung sind erfindungsgemäß auch diagnostische Anwendungen auf FKBP1A gerichtet. Da FKBP1A im Gegensatz zum gesunden Brustgewebe stark in Triple-negativen Brustkrebszellen exprimiert wird, besteht ein hohes Potential, über ein geeignetes Testverfahren FKBP1A im Gewebe und Serum bzw. Plasma von Patienten sowohl für die Primärdiagnostik als auch therapeutische Verlaufskontrolle zu verwenden. Darüber hinaus wird FKBP1A stärker in "mesenchymal stem-like" triple-negativen Brustkrebszellen, für welche ein besonders hoher Stammzellcharakter beschrieben ist, als in basal-like triple-negativen Brustkrebszellen exprimiert (siehe Abbildung 2). Daher könnte FKBP1A dabei ein diagnostischer und prognostischer Marker für triple-negative Brustkrebszellen mit einer besonders stark epigenetisch deregulierten, geringen Zelldifferenzierung, geringen epithelialen Charakter und hohen Aggressivität von triple-negativen Tumoren sein. Aufgrund der hohen Korrelation zu EMT-Markern kann FKBP1A zudem als diagnostischer und prognostischer Marker in Tumorzellen der Tumor-Extrazellulär-Matrix für invasierende Tumore verwendet werden.

Abbildung 1 zeigt eine Western-Blot Analyse der Proteinexpression von FKBP1A in verschiedenen Zellen.

Abbildung 2 zeigt die Herabregulation der Proteinexpression von FKBP1A durch spezifische siRNA als Ergebnis einer Western-Blot Analyse.

Abbildung 3 zeigt das Ergebnis einer Expressionsanalyse von (Tumor-)Stammzell- und mesenchymalen Markern nach FKBP1A knock-down.

Abbildung 4 zeigt die Herabregulation der Proteinexpression von FKBP1A durch Inhibitor (I) als Ergebnis einer Western-Blot Analyse der Proteinexpression.

Abbildung 5 zeigt das Ergebnis einer Expressionsanalyse von (Tumor-)Stammzell- und epithelialen Differenzierungsmarkern sowie PD-L1 nach Expressionsreduktion von FKBP1A mittels Inhibitor (I).

Abbildung 6 zeigt bekannte Interaktionspartner von FKBP1A (Quelle: canSAR Black (https://cansarblack.icr.ac.uk/?type=protein,compound,cellline,disease).

Abbildung 7 zeigt ein Beispiel für den theoretischen Aufbau eines dual-targeting Wirkstoffs gegen FKBP1A und PIN1.

Abbildung 8 zeigt auf Abbildung 7 aufbauende Beispiele für aus Rapamycin und KPT-6556 aufgebaute dual-targeting Wirkstoffe.

Abbildung 9 zeigt auf Abbildung 7 aufbauende Beispiele für aus Rapamycin und ATRA aufgebaute dual-targeting Wirkstoffe.

Abbildung 10 zeigt eine Ko-Expressionsanalyse von FKBP1A und TGF-beta-Rezeptor II in primären Brustkrebsgewebe.

Ein Aspekt der Erfindung betrifft ein Verfahren zum Auffinden von Inhibitoren der Peptidyl-prolyl cis-trans Isomerase FKBP1A, welche zur Behandlung des Triple-negativen Mammakarzinoms geeignet sind, umfassend die Schritte
(a) Bereitstellen eines Inhibitors
   (i) der Expression von FKBP1A; und/oder
   (ii) der enzymatischen Aktivität von FKBP1A; und/oder
   (iii) der Interaktion(en) von FKBP1A mit Interaktionspartner(n);
(b) Inkubieren von Krebszellen einer Krebszelllinie mit dem Inhibitor von FKBP1A, und Ermitteln einer Eigenschaft der Krebszellen ausgewählt aus der Gruppe bestehend aus (A) zellulärem Differenzierungsstatus, (B) epithelialen Eigenschaften, (C) zellulärer Immunogenität, (D) Apoptoseinduzierung bzw. Apoptosefähigkeit, und (E) zellulärem Stammzellcharakter;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b);
(d) Vergleichen der ermittelten Eigenschaft der Krebszellen gemäß Schritten (b) und (c).

Bevorzugt umfasst Schritt (a) des erfindungsgemäßen Verfahrens die Teilschritte
(a₁) Bereitstellen mehrerer Testsubstanzen;
(a₂) Durchmustern der Testsubstanzen hinsichtlich ihrer inhibierenden Wirkung auf
   (i) die Expression von FKBP1A; und/oder
   (ii) die enzymatische Aktivität von FKBP1A; und/oder
   (iii) Interaktion(en) von FKBP1A mit Interaktionspartner(n);
(a₃) Auswählen mindestens einer durchmusterten Testsubstanz, deren inhibierende Wirkung stärker ist als die inhibierende Wirkung mindestens einer anderen durchmusterten Testsubstanz, und Bereitstellen dieser ausgewählten Testsubstanz als Inhibitor von FKBP1A.

Typischerweise ist die Krebszelllinie charakteristisch für das Triple-negative Mammakarzinom oder ist eine Krebszelllinie des Triple-negativen Mammakarzinoms, bevorzugt ausgewählt aus der Gruppe bestehend aus MDA-MB-231, MDA-MB-436 und MDA-MB-468; insbesondere MD-MB-231. Geeignete Krebszelllinien sind z.B. erhältlich über die American Type Culture Collection.

Die in Schritt (b) ermittelte Eigenschaft der Krebszellen ist ausgewählt aus der Gruppe bestehend aus (A) zellulärem Differenzierungsstatus, (B) epithelialen Eigenschaften, (C) zellulärer Immunogenität, (D) Apoptoseinduzierung bzw. Apoptosefähigkeit, und (E) zellulärem Stammzellcharakter.

In bevorzugten Ausführungsformen des erfindungsgemäßen Verfahrens inhibiert der in Schritt (a) bereitgestellte Inhibitor die Interaktion(en) von FKBP1A mit Interaktionspartner(n), wobei mindestens ein Interaktionspartner ausgewählt ist aus der Gruppe bestehend aus Interaktionspartnern, welche eine Reduktion oder Arretierung (A) des zellulären Differenzierungsstatus', (B) der epithelialen Eigenschaften, (C) der zellulären Immunogenität, (D) der Apoptoseinduzierung bzw. Apoptosefähigkeit und/oder (E) des zellulären Stammzellcharakters bewirken.

In bevorzugten Ausführungsformen des erfindungsgemäßen Verfahrens inhibiert der in Schritt (a) bereitgestellte Inhibitor die Interaktion(en) von FKBP1A mit Interaktionspartner(n), wobei mindestens ein Interaktionspartner ausgewählt ist aus der Gruppe bestehend aus interagierenden Proteinen, interagierenden Peptiden, und interagierenden Nukleinsäuren.

Zahlreiche Interaktionspartner von FKBP1A sowie deren Wechselwirkungen sind einem Fachmann bekannt. Erfindungsgemäß bevorzugte Interaktionspartner von FKBP1A sind ausgewählt aus der Gruppe bestehend aus AHSP, Cyclophilin-Typ Peptidyl-Prolyl Isomerasen, FKBP-Typ Peptidyl-Prolyl Isomerasen, Inositoltriphosphat Rezeptoren, MDM2/MDM4, Phosphoprotein Phosphatasen, Ryanodin-Rezeptoren, SF3B4, TGF-beta, TKL Ser/Thr Proteinkinasen und Typ-B Carboxylesterasen/ Lipasen. Erfindungsgemäß bevorzugte Interaktionspartner von FKBP1A sind in nachfolgender Tabelle zusammengefasst (vgl. Abbildung 6):

| Name | Uniprot ID | Familie |
|---|---|---|
| ACVR1B | P36896 | TKL Ser/Thr Proteinkinasen (*tyrosine kinase like*) |
| ACVRL1 | P37023 | TKL Ser/Thr Proteinkinasen (*tyrosine kinase like*) |
| AHSP | Q9NZD4 | AHSP (*alpha-hemoglobin-stabilizing protein*) |
| BARD1 | Q99728 | |
| BMPR1A | P36894 | TKL Ser/Thr Proteinkinasen (*tyrosine kinase like*) |
| FKBP1A | P62942 | FKBP-Typ Peptidyl-Prolyl Isomerasen (*FK506 binding protein*) |
| FKBP4 | Q02790 | |
| GLMN | Q92990 | |
| ITPR1 | Q14643 | Inositoltriphosphat (InsP3) Rezeptoren |
| MDM2 | Q00987 | MDM2/MDM4 (*murine double minute 2*/*human homolog*) |
| NLGN3 | Q9NZ94 | Typ-B Carboxylesterasen/Lipasen |
| PPIA | P62937 | Cyclophilin-Typ Peptidyl-Prolyl Isomerasen |
| PPP3CA | Q08209 | Phosphoprotein Phosphatasen |
| RYR3 | Q15413 | Ryanodin-Rezeptoren (TC 1.A.3.1) |
| SF3B4 | Q15427 | SF3B4 (*splicing factor 3B subunit 4*) |
| TGFB1 | P01137 | TGF-beta (*transforming growth factor beta*) |
| TGFBR1 | P36897 | TKL Ser/Thr Proteinkinasen (*tyrosine kinase like*) |
| TRPC3 | Q13507 | TRP Rezeptoren (TC 1.A.4) (*tryptophan transient receptor*) |

In bevorzugten Ausführungsformen des erfindungsgemäßen Verfahrens wird in Schritt (b) die Eigenschaft der Krebszellen ermittelt durch Quantifizierung von Differenzierungsmarkern (Zelltyp-Marker).

Geeignete Differenzierungsmarker und Methoden für deren jeweilige Quantifizierung sind einem Fachmann bekannt. In diesem Zusammenhang kann beispielsweise vollumfänglich verwiesen werden auf: Akrap, N. *et al.* Identification of Distinct Breast Cancer Stem Cell Populations Based on Single-Cell Analyses of Functionally Enriched Stem and Progenitor Pools. *Stem Cell Reports* (2016)*.* doi:10.1016/j.stemcr.2015.12.006; Prabhakaran, P., Hassiotou, F., Blancafort, P. & Filgueira, L. Cisplatin induces differentiation of breast cancer cells. Front. Oncol. (2013). doi:10.3389/fonc.2013.00134.

Geeignete Differenzierungsmarker sind z.B. CD3 auf T-Lymphozyten, CD14 auf Monozyten, CD16 und CD56 auf NK-Zellen und Granulozyten, CD19 und CD20 auf B-Lymphozyten. Differenzierungsmarker für mesenchymale Stammzellen sind z.B. CD13, CD29, CD44, CD49e, CD54, CD71, CD73, CD90, CD105, CD106, CD166 und HLA-ABC. Bevorzugt sind die Differenzierungsmarker solche von Epithelzellen.

In bevorzugten Ausführungsformen des erfindungsgemäßen Verfahrens wird in Schritt (b) die Eigenschaft der Krebszellen ermittelt durch Quantifizierung von Epithelmarkern.

Geeignete Epithelmarker und Methoden für deren jeweilige Quantifizierung sind einem Fachmann bekannt. In diesem Zusammenhang kann beispielsweise vollumfänglich verwiesen werden auf Frixen, U. H. et al. E-cadherin-mediated cell-cell adhesion prevents invasiveness of human carcinoma cells. J. Cell Biol. (1991). doi:10.1083/jcb.113.1.173.

Erfindungsgemäß bevorzugte Epithelmarker (Epithelzellen-Marker) sind ausgewählt aus der Gruppe bestehend aus Zytokeratinen, Zelladhäsionsmolekülen und Zelloberflächenproteinen. Ein erfindungsgemäß bevorzugtes Zelladhäsionsmolekül, welches als Epithelmarker eingesetzt werden kann, ist E-Cadherin.

In bevorzugten Ausführungsformen des erfindungsgemäßen Verfahrens wird in Schritt (b) die Eigenschaft der Krebszellen ermittelt durch Quantifizierung von Mesenchymalmarkern.

Geeignete Mesenchymalmarker und Methoden für deren jeweilige Quantifizierung sind einem Fachmann bekannt. In diesem Zusammenhang kann beispielsweise vollumfänglich verwiesen werden auf Yamashita, N. et al. Vimentin as a poor prognostic factor for triple-negative breast cancer. J. Cancer Res. Clin. Oncol. (2013). doi:10.1007/s00432-013-1376-6.

Erfindungsgemäß bevorzugte Mesenchymalmarker sind Vimentin, Fibronectin und N-Cadherin (Ogunbolude, Y. et al. FRK inhibits breast cancer cell migration and invasion by suppressing Epithelial-mesenchymal transition. Oncotarget (2017). doi:10.18632/oncotarget.22958).

Erfindungsgemäß bevorzugte Mesenchymalmarker und Marker für EMT sind Vimentin, Fibronectin, N-Cadherin, SNAI1, TWIST1. TWIST2, ZEB1 und ZEB2 (Ogunbolude, Y. et al. FRK inhibits breast cancer cell migration and invasion by suppressing Epithelial-mesenchymal transition. Oncotarget (2017). doi:10.18632/oncotarget.22958).

In bevorzugten Ausführungsformen des erfindungsgemäßen Verfahrens wird in Schritt (b) die Eigenschaft der Krebszellen ermittelt durch Quantifizierung von (Tumor-)Stammzellmarkern.

Geeignete (Tumor-)-Stammzellmarker und Methoden für deren jeweilige Quantifizierung sind einem Fachmann bekannt. In diesem Zusammenhang kann beispielsweise vollumfänglich verwiesen werden auf Li, W. et al. Unraveling the roles of CD44/CD24 and ALDH1 as cancer stem cell markers in tumorigenesis and metastasis. Sci. Rep. (2017). doi:10.1038/s41598-017-14364-2.

Erfindungsgemäß bevorzugte (Tumor-)Stammzellmarker sind CD44/CD24 und ALDH1.

In bevorzugten Ausführungsformen des erfindungsgemäßen Verfahrens wird in Schritt (b) die Eigenschaft der Krebszellen ermittelt durch Quantifizierung von immunmodulierenden Proteinen.

Geeignete immunmodulierende Proteine und Methoden für deren jeweilige Quantifizierung sind einem Fachmann bekannt. In diesem Zusammenhang kann beispielsweise vollumfänglich verwiesen werden auf Bu, X., Yao, Y. & Li, X. Immune checkpoint blockade in breast cancer therapy. in Advances in Experimental Medicine and Biology (2017). doi:10.1007/978-981-10-6020-5_18.

Erfindungsgemäß bevorzugte immunmodulierende Proteine sind Check-Point-Proteine, z.B. PD-L1, PD-L2; (ggf. in Ko-Kultur mit T-Zellen): PD-1 und CTLA-4.

In bevorzugten Ausführungsformen des erfindungsgemäßen Verfahrens wird in Schritt (b) die Eigenschaft der Krebszellen ermittelt durch Quantifizierung von immunvermittelnden Proteinen.

Geeignete immunvermittelnde Proteine und Methoden für deren jeweilige Quantifizierung sind einem Fachmann bekannt. In diesem Zusammenhang kann beispielsweise vollumfänglich verwiesen werden auf Vertuani, S. et al. Retinoids Act as Multistep Modulators of the Major Histocompatibility Class I Presentation Pathway and Sensitize Neuroblastomas to Cytotoxic Lymphocytes. Cancer Res. 63, 8006-8013 (2003).

Ein erfindungsgemäß bevorzugtes immunvermittelndes Protein ist der MHC Klasse I Proteinkomplex.

In bevorzugten Ausführungsformen des erfindungsgemäßen Verfahrens umfasst Schritt (b) das Inkubieren einer Mischung von Krebszellen einer Krebszelllinie und Immunzellen einer Immunzelllinie mit dem Inhibitor von FKBP1A, wobei als Eigenschaft der Krebszellen deren zelluläre Immunogenität ermittelt wird durch Quantifizierung der Immunzellaktivierung und/oder durch Quantifizierung der immunzellvermittelten zytotoxischen Wirkung auf die Krebszellen.

Geeignete Methoden zur Quantifizierung der Immunzellaktivierung und zur Quantifizierung der immunzellvermittelten zytotoxischen Wirkung auf die Krebszellen sind einem Fachmann bekannt.

Gemäß dieser bevorzugten Ausführungsform umfasst Schritt (c) des erfindungsgemäßen Verfahrens das Inkubieren einer Mischung von Krebszellen der gleichen Krebszelllinie und Immunzellen der gleichen Immunzelllinie wie bei Schritt (b) in Abwesenheit des Inhibitors von FKBP1A und das Ermitteln der gleichen Eigenschaft der Krebszellen wie bei Schritt (b) unter gleichen Bedingungen wie Schritt (b).

In bevorzugten Ausführungsformen des erfindungsgemäßen Verfahrens inhibiert der in Schritt (a) bereitgestellte Inhibitor (i) die Expression von FKBP1A und nicht gleichzeitig (ii) die enzymatische Aktivität von FKB1A.

In anderen bevorzugten Ausführungsformen des erfindungsgemäßen Verfahrens inhibiert der in Schritt (a) bereitgestellte Inhibitor (ii) die enzymatische Aktivität von FKB1A und gleichzeitig nicht (i) die Expression von FKBP1A.

In bevorzugten Ausführungsformen umfasst das erfindungsgemäße Verfahren die Schritte
(a) Bereitstellen eines Inhibitors (i) der Expression von FKBP1A;
(b) Inkubieren von Krebszellen einer Krebszelllinie, bevorzugt einer Krebszelllinie, welche für das Triple-negative Mammakarzinom charakteristisch ist, mit dem Inhibitor von FKBP1A, und Ermitteln einer Eigenschaft der Krebszellen;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b); und
(d) Vergleichen der ermittelten Eigenschaft der Krebszellen gemäß Schritten (b) und (c).

Gemäß dieser bevorzugten Ausführungsform umfasst Schritt (a) die Teilschritte
(a₁) Bereitstellen mehrerer Testsubstanzen;
(a₂) Durchmustern der Testsubstanzen hinsichtlich ihrer inhibierenden Wirkung auf (i) die Expression von FKBP1A; und
(a₃) Auswählen mindestens einer durchmusterten Testsubstanz, deren inhibierende Wirkung stärker ist als die inhibierende Wirkung mindestens einer anderen durchmusterten Testsubstanz, und Bereitstellen dieser ausgewählten Testsubstanz als Inhibitor von FKBP1A.

In bevorzugten Ausführungsformen des erfindungsgemäßen Verfahrens ist die Krebszelllinie charakteristisch ist für das Triple-negative Mammakarzinom; bevorzugt ist die Krebszelllinie eine Krebszelllinie des Triple-negativen Mammakarzinoms.

In bevorzugten Ausführungsformen des erfindungsgemäßen Verfahrens ist die Krebszelllinie eine Krebszelllinie des Triple-negativen Mammakarzinoms vom Subtyp mesenchymal stem-like. Bevorzugt ist die Krebszelllinie des Triple-negativen Mammakarzinoms vom Subtyp mesenchymal stem-like ausgewählt aus MDA-MB-231 und MDA-MB-436; bevorzugt MDA-MB-231.

In bevorzugten Ausführungsformen des erfindungsgemäßen Verfahrens ist die Krebszelllinie eine Krebszelllinie des Triple-negativen Mammakarzinoms vom Subtyp basal-like. Bevorzugt ist die Krebszelllinie des Triple-negativen Mammakarzinoms vom Subtyp basal-like MDA-MB-468.

In bevorzugten Ausführungsformen des erfindungsgemäßen Verfahrens ist die in Schritt (b) ermittelte Eigenschaft (A) zellulärer Differenzierungsstatus. Gemäß dieser bevorzugten Ausführungsform des erfindungsgemäßen Verfahrens wird in Schritt (b) die Eigenschaft bevorzugt durch Quantifizierung von Mesenchymalmarkern ermittelt; bevorzugt durch Quantifizierung von Vimentin.

In weiteren bevorzugten Ausführungsformen des erfindungsgemäßen Verfahrens ist die in Schritt (b) ermittelte Eigenschaft (C) zelluläre Immunogenität. Gemäß dieser bevorzugten Ausführungsform des erfindungsgemäßen Verfahrens wird in Schritt (b) die Eigenschaft bevorzugt durch Quantifizierung von immunmodulierenden Proteinen ermittelt; bevorzugt durch Quantifizierung von PD-L1.

In weiteren bevorzugten Ausführungsformen des erfindungsgemäßen Verfahrens ist die in Schritt (b) ermittelte Eigenschaft (E) zellulärer Stammzellcharakter. Gemäß dieser bevorzugten Ausführungsform des erfindungsgemäßen Verfahrens wird in Schritt (b) die Eigenschaft bevorzugt durch Quantifizierung von (Tumor-)Stammzellmarkern ermittelt; bevorzugt durch Quantifizierung von CD44/CD24 und ALDH1.

In weiteren bevorzugten Ausführungsformen des erfindungsgemäßen Verfahrens ist die in Schritt (b) ermittelte Eigenschaft die Quantifizierung der Expression von FKBP1A. Geeignete Methoden zur Quantifizierung der Expression von FKBP1A sind einem Fachmann bekannt, z.B. die Western-Blot-Analyse.

In bevorzugten Ausführungsformen umfasst das erfindungsgemäße Verfahren die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt den zellulären Differenzierungsstatus, bevorzugter durch Quantifizierung von Mesenchymalmarkern;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b); und
(d) Vergleichen der ermittelten Eigenschaft der Krebszellen gemäß Schritten (b) und (c).

In bevorzugten Ausführungsformen umfasst das erfindungsgemäße Verfahren die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt zelluläre Immunogenität, bevorzugter durch Quantifizierung von immunmodulierenden Proteinen;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b); und
(d) Vergleichen der ermittelten Eigenschaft der Krebszellen gemäß Schritten (b) und (c).

In bevorzugten Ausführungsformen umfasst das erfindungsgemäße Verfahren die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt zellulärer Stammzellcharakter, bevorzugter durch Quantifizierung von (Tumor-)Stammzellmarkern;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b); und
(d) Vergleichen der ermittelten Eigenschaft der Krebszellen gemäß Schritten (b) und (c).

In bevorzugten Ausführungsformen umfasst das erfindungsgemäße Verfahren die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt die Quantifizierung der Expression von FKBP1A;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b); und
(d) Vergleichen der ermittelten Eigenschaft der Krebszellen gemäß Schritten (b) und (c).

Gemäß dieser bevorzugten Ausführungsform umfasst Schritt (a) die Teilschritte
(a₁) Bereitstellen mehrerer Testsubstanzen;
(a₂) Durchmustern der Testsubstanzen hinsichtlich ihrer inhibierenden Wirkung auf (i) die Expression von FKBP1A;
(a₃) Auswählen mindestens einer durchmusterten Testsubstanz, deren inhibierende Wirkung stärker ist als die inhibierende Wirkung mindestens einer anderen durchmusterten Testsubstanz, und Bereitstellen dieser ausgewählten Testsubstanz als Inhibitor von FKBP1A.

Ein weiterer nicht wörtlich beanspruchter Aspekt der Erfindung betrifft die Verwendung eines
(A) Inhibitors
   (i) der Expression von FKBP1A; und/oder
   (ii) der enzymatischen Aktivität von FKBP1A; und/oder
   (iii) der Interaktion(en) von FKBP1A mit Interaktionspartner(n); und/oder
(B) PROTACs (PROteolysis TArgeting Chimeras), welches spezifisch den zellulären Abbau von FKBP1A induziert, bevorzugt FKBP12 PROTAC RC32 (CAS No.: 2375555-66-9),
zur Herstellung eines Medikaments zur Behandlung einer Erkrankung; insbesondere Krebserkrankung; bevorzugt des Triple-negativen Mammakarzinoms, des Her2-positiven Hormonrezeptor-negativen Mammakarzinoms, des duktalen Adenokarzinoms des Pankreas (PDAC) oder des malignen Melanoms; besonders bevorzugt von sog. Tumorstammzellen; besonders bevorzugt des Triple-negativen Mammakarzinoms, bevorzugt des mesenchymal stem-like Subtyps.

In einer bevorzugten Ausführungsform inhibiert der Inhibitor (i) die Expression von FKBP1A und gleichzeitig nicht (ii) die enzymatische Aktivität von FKB1A.

In einer bevorzugten Ausführungsform inhibiert der Inhibitor (ii) die enzymatische Aktivität von FKB1A und gleichzeitig nicht (i) die Expression von FKBP1A.

Erfindungsgemäße Therapien basieren somit auf verschiedenen Ansätzen, greifen insbesondere in verschiedene Stadien der Bildung und Wirkung von FKBP1A in Tumorzellen ein. Die Inhibierung der Expression von FKBP1A verfolgt das Ziel, dass FKBP1A gar nicht erst gebildet wird oder dessen Konzentration zumindest reduziert wird. Die Inhibierung der enzymatischen Funktion von FKBP1A verfolgt das Ziel, exprimierte FKBP1A daran zu hindern, die enzymatisch-katalysierte Wirkung zu entfalten. Die Inhibierung der Interaktion(en) von FKBP1A mit Interaktionspartner(n) verfolgt das Ziel, Sekundärprozesse zu unterdrücken.

In einer bevorzugten Ausführungsform wird ein Inhibitor der Expression von FKBP1A zur therapeutischen Behandlung der Erkrankung verwendet.

In bevorzugten Ausführungsformen der Erfindung ist der Inhibitor der Expression von FKBP1A eine FKBP1A-spezifische siRNA.

Geeignete siRNA, welche für FBBP1A spezifisch ist, ist einem Fachmann bekannt und kommerziell erhältlich, beispielsweise siRNA S5205 (ThermoFisher Scientific, Waltham, USA).

In einer bevorzugten Ausführungsform wird ein Inhibitor der enzymatischen Aktivität von FKBP1A zur therapeutischen Behandlung der Erkrankung verwendet.

In bevorzugten Ausführungsformen der Erfindung ist der Inhibitor der enzymatischen Aktivität von FKBP1A FK506 oder ein spezifischer nicht-immunsuppressiver Inhibitor.

In bevorzugten Ausführungsformen der Erfindung ist der Inhibitor der enzymatischen Aktivität von FKBP1A ein spezifischer nicht-immunsuppressiver, isoliert das Enzym adressierende Inhibitor. Bevorzugt ist der Inhibitor ein spezifischer Inhibitor von FKBP1A ohne mTOR- bzw. Calcineurin-Bindung und immun-suppressive Wirkung.

In einer bevorzugten Ausführungsform wird ein Inhibitor der Interaktion(en) von FKBP1A mit Interaktionspartner(n) zur therapeutischen Behandlung der Erkrankung verwendet.

Erfindungsgemäß bevorzugte Interaktionspartner von FKBP1A sind auf hinsichtlich dieses Aspekts der Erfindung bevorzugt ausgewählt aus der Gruppe bestehend aus AHSP, Cyclophilin-Typ Peptidyl-Prolyl Isomerasen, FKBP-Typ Peptidyl-Prolyl Isomerasen, Inositoltriphosphat Rezeptoren, MDM2/MDM4, Phosphoprotein Phosphatasen, Ryanodin-Rezeptoren, SF3B4, TGF-beta, TKL Ser/Thr Proteinkinasen und Typ-B Carboxylesterasen/ Lipasen.

Bevorzugt erfolgt die erfindungsgemäße therapeutische Behandlung als adjuvante Behandlung einer Krebserkrankung. Dabei erfolgt die erfindungsgemäße therapeutische Behandlung bevorzugt als adjuvante Behandlung bei einer Immuntherapie der Krebserkrankung und/oder zur Vermeidung und/oder gezielten Adressierung von invasierenden Tumorzellen, zur Vermeidung von Tumorzell-Invasion und Metastasenbildung.

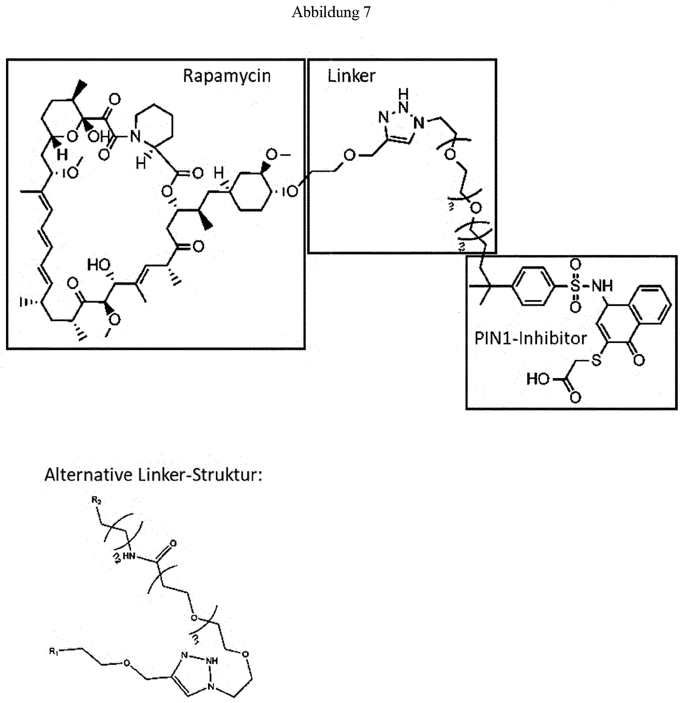

Eine besondere Therapieform zur gezielten Behandlung des Triple-negativen Mammakarzinoms ist die simultane Adressierung von FKBP1A und des Parvulins PIN1 oder alternativ der Retinsäure-Rezeptoren RAR bzw. RXR. Es konnte hier erstmals eine starke synergistische Wirkung von Inhibition von FKBP1A und Inhibition von PIN1 (hier mittels FKBP1A-spezifischer siRNA (oder alternativ eines FKBP1A-spezifischen Inhibitors) und Koinkubation mit dem PIN1-spezifischem Inhibitor all-trans-Retinsäure, ATRA) beim Triple-negativen Mammakarzinom auf u.a. die Reduktion des Tumorstammzellcharakters und Apoptoseinduzierbarkeit gezeigt werden. Eine solche Therapie kann zum einen über die kombinierte Applikation einzelner spezifischer Wirkstoffe erreicht werden. In diesem Zusammenhang kann zum anderen über das Konzept der zukunftsweisenden Polypharmakologie ein Dual-targeting Inhibitor entwickelt werden, welcher als einzelner Wirkstoff gezielt beide Peptidyl-Prolyl-cis-trans-Isomerasen adressiert. Dieser könnte z.B. aus jeweils spezifischen Inhibitoren wie z.B. ElteN378, V-10367 oder GPI-1046 gegen FKBP1A auf der einen Seite und z.B. KPT-6566, ATRA (all-trans-Retinsäure) oder eines ATRA-Derivates gegen PIN1, RAR oder RXR (Chen, Y. et al. Prolyl isomerase Pin1: a promoter of cancer and a target for therapy, Cell Death and Disease (2018), doi:10.1038/s41419-018-0844-y) auf der anderen Seite aufgebaut sein, die beide entweder fusioniert oder über einen Linker verbunden sind, oder bei dem funktionelle Teilstrukturen der Inhibitoren "gemergt" sind (Ramsay, R. R., Popovic-Nikolic, M. R., Nikolic, K., Uliassi, E. & Bolognesi, M. L. A perspective on multi-target drug discovery and design for complex diseases, Clin. Transl. Med. (2018), doi:10.1186/s40169-017-0181-2). Als chemisches Beispiel, in dem ein FKBP1A-Ligand an einen zweiten Inhibitor/Liganden gekoppelt wurde, dienen die Wirkstoffe RapaLink-01 (CAS No.: 1887095-82-0) oder FKBP12 PROTAC RC32 (CAS No.: 2375555-66-9). Ein hierzu vergleichbarer Wirkstoff lässt sich von einem Experten mit einem PIN1-Inhibitor wie z.B. KPT-6566 oder ATRA (all-trans-Retinsäure) oder einem ATRA-Derivat entwickeln und synthetisieren. Weitere Linker-Strukturen von FKBP1A-Liganden sind bekannt (Kolos, J. M., Voll, A. M., Bauder, M. & Hausch, F. FKBP Ligands - Where We Are and Where to Go? Front. Pharmacol. (2018), doi:10.3389/fphar.2018.01425) und lassen sich ebenfalls von einem Experten mit einem PIN1-Inhibitor wie z.B. KPT-6566 oder ATRA (all-trans-Retinsäure) oder einem ATRA-Derivat zu einem dual-targeting Wirkstoff entwickeln. Beispielhafte Strukturformeln für die genannten dual-targeting Wirkstoffe sind in den Abbildungen 7 bis 9 aufgeführt. Bevorzugt entspricht der Wirkstoff gemäß Unteranspruch 13 einem der in den Abbildungen 7 bis 9 aufgeführten Wirkstoffe.

Abbildung 7 zeigt ein Beispiel für den theoretischen Aufbau eines dual-targeting Wirkstoffs gegen FKBP1A und PIN1. Der linke Kasten zeigt die Strukturkomponente entsprechend dem Rapamycin, der mittlere Kasten zeigt eine exemplarische Linker-Struktur und der rechte Kasten einen exemplarischen PIN1-Inhibitor. Alternativ zu der im mittleren Kasten angegebenen Linker-Struktur kann auch die alternative Linker-Struktur verwendet werden, wobei R₁ die Rapamycin- und R₂ der PIN1-Inhibitor-Struktur darstellen. Der Wert für n₁ bzw. n₂ ist jeweils ein beliebiger positiver Wert inklusive dem Wert 0.

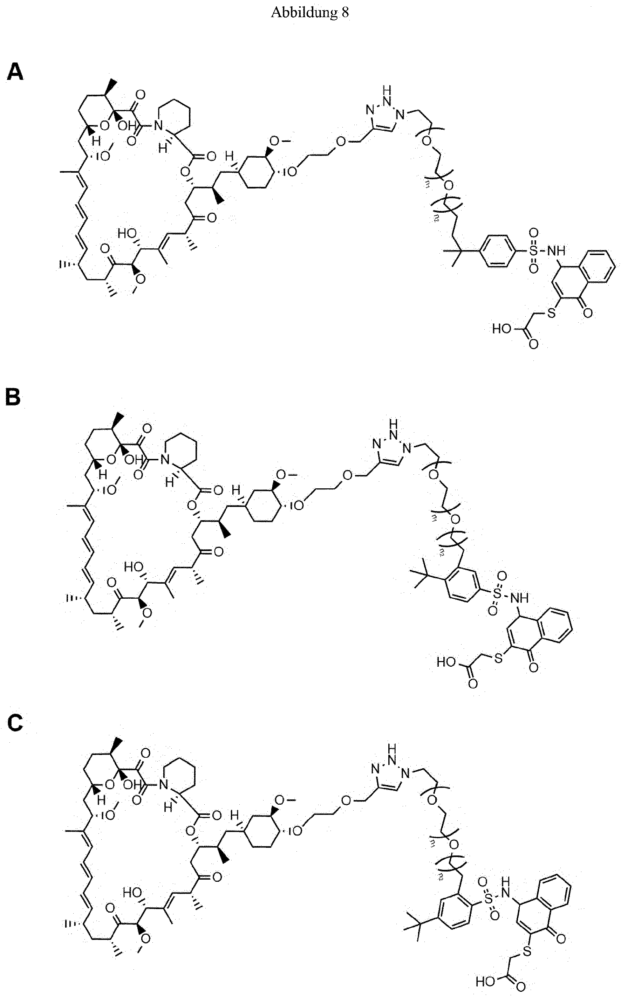

Abbildung 8 zeigt auf Abbildung 7 aufbauende Beispiele für aus Rapamycin und KPT-6556 aufgebaute dual-targeting Wirkstoffe. Die chemische Bindung des Linkers an KPT-6556 bzw. Rapamycin ist auch an alle anderen möglichen Atome der jeweiligen Teilstrukturen denkbar und von einem Experten durchführbar. Der Wert für n₁ bzw. n₂ ist jeweils ein beliebiger positiver Wert inklusive dem Wert 0.

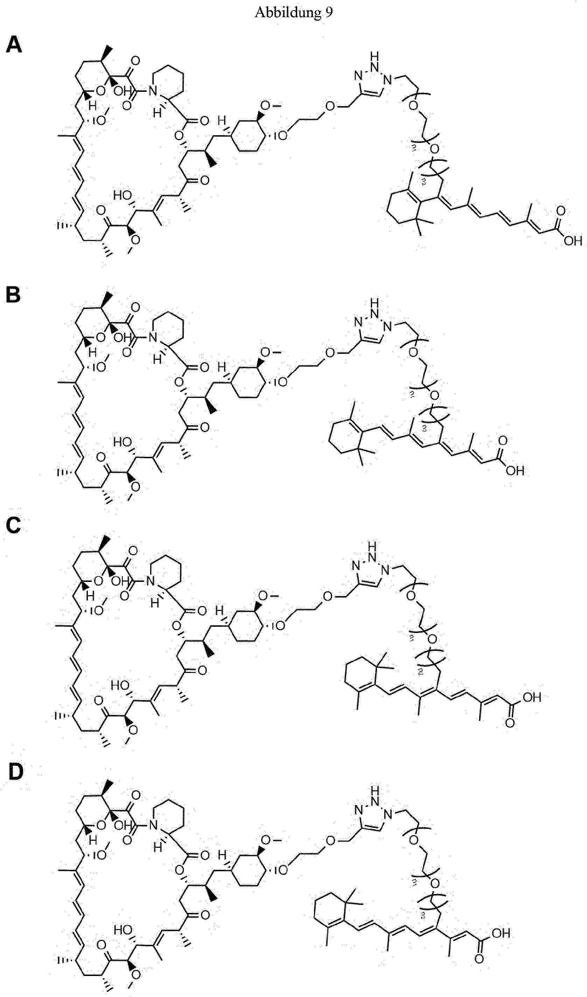

Abbildung 9 zeigt auf Abbildung 7 aufbauende Beispiele für aus Rapamycin und ATRA aufgebaute dual-targeting Wirkstoffe. Die chemische Bindung des Linkers an ATRA bzw. Rapamycin ist auch an alle anderen möglichen Atome der jeweiligen Teilstrukturen denkbar und von einem Experten durchführbar. Der Wert für n₁ bzw. n₂ ist jeweils ein beliebiger positiver Wert inklusive dem Wert 0.

Die unter [0062] bis [0065] beschriebene chemische Bindung eines PIN1-spezifischen Wirkstoffes als dual-targeting Wirkstoff an einen hochaffinen Liganden von FKBP1A wie z.B. Rapamycin und die hierüber bewirkte intrazelluläre Bindung des PIN1-spezifischen Wirkstoffes an FKBP1A kann auch für beliebig andere Wirkstoffe erfolgen. Die Kopplung an das cytosolische Protein FKBP1A hat dabei wesentliche Änderungen auf die Pharmakokinetik und -dynamik des beliebigen Wirkstoffes: In Tumorzellen, insbesondere Tumorstammzellen, sind verschiedene Transporter, u.a. ABC-Transporter, stark überexprimiert (Choi, Y. & Yu, A.-M. ABC Transporters in Multidrug Resistance and Pharmacokinetics, and Strategies for Drug Development. Curr. Pharm. Des. (2014). doi:10.2174/138161282005140214165212). Diese Transporter sorgen über einen verstärkten Efflux der Wirkstoffe für einen schnellen Abtransport der Wirkstoffe aus der Zelle, so dass diese nur bedingt und kurzfristig intrazellular eine Wirkung entfalten können. Hinzu kommen Metabolisierungen von Wirkstoffen in der Zelle, die ebenfalls zu Wirkungsverlusten führen. Eine chemische Kopplung wie unter [0062] bis [0065] beschrieben würde den beliebigen Wirkstoff an das cytosolische und dadurch frei in der Zelle diffundierende Protein FKBP1A binden. Der gebildete Komplex wird jedoch u.a. aufgrund seiner Größe im Gegensatz zum einzelnen Wirkstoff nicht oder wesentlich weniger über die genannten Transporter aus der Zelle befördert oder durch zelluläre Prozesse metabolisiert, worüber eine wesentlich bessere intrazelluläre Wirkung des Wirkstoffes erreicht werden kann. Die chemische Bindung eines beliebigen Wirkstoffes an einen hochaffinen Liganden von FKBP1A oder von einem anderen cytosolischen Protein kann zukünftig als allgemeines neuartiges pharmakologisches Prinzip mit dem Ziel u.a. einer Wirkungsverstärkung eines beliebigen Wirkstoffes in der Zelle insbesondere in Tumorstammzellen oder resistenten Tumorzellen eingeführt werden.

Die erfindungsgemäße therapeutische Behandlung erfolgt als Behandlung von Erkrankungen, welche bevorzugt ausgewählt sind aus Krebserkrankungen und neurodegenerativen Erkrankungen wie z.B. Morbus Alzheimer.

Bevorzugt erfolgt die erfindungsgemäße therapeutische Behandlung als Behandlung von Krebserkrankungen, welche ausgewählt sind aus FKBP1A-positiven Tumoren. Besonders bevorzugt ist die Krebserkrankung ausgewählt aus Triple-negativem Mammakarzinom, Her2-positivem Hormonrezeptor-negativem Mammakarzinom und malignem Melanom; insbesondere Triple-negatives Mammakarzinom, ganz insbesondere des TNBC-mesenchymal stem-like Subtyps.

Ein weiterer nicht wörtlich beanspruchter Aspekt der Erfindung betrifft die Verwendung von FKBP1A als prognostischer oder diagnostischer Marker von Erkrankungen, insbesondere Krebserkrankungen oder neurodegenerative Erkrankungen; insbesondere FKBP1A-positiver Tumore; insbesondere des Triple-negativen Mammakarzinoms, des Her2-positiven Hormonrezeptor-negativen Mammakarzinoms oder des malignen Melanoms; insbesondere des Triple-negativen Mammakarzinoms, ganz insbesondere des TNBC-mesenchymal stem-like Subtyps.

Ein weiterer nicht wörtlich beanspruchter Aspekt der Erfindung betrifft ein Verfahren zur quantitativen Detektion von FKBP1A in humanem Material zur prognostischen und diagnostischen Beurteilung von Erkrankungen; insbesondere Krebserkrankungen; bevorzugt Krebserkrankungen mit hohem Metastasierungpotential inkl. zur Analyse und Diagnose von Tumor-Invasion und zur Vorhersage einer Metastasierungstendenz; insbesondere des Triple-negativen Mammakarzinoms, des Her2-positiven Hormonrezeptor-negativen Mammakarzinoms oder des malignen Melanoms; insbesondere des Triple-negativen Mammakarzinoms, ganz insbesondere des TNBC-mesenchymal stem-like Subtyps; umfassend die Schritte:
(a) Bereitstellen von humanem Material, bevorzugt Serum, Plasma oder Gewebe; und
(b) Quantifizieren von FKBP1A, bevorzugt durch Konzentrationsbestimmung.

Bevorzugt beinhaltet Schritt (a) des erfindungsgemäßen Verfahrens zur quantitativen Detektion von FKBP1A in humanem Material das Bereitstellen des humanen Materials, aber nicht die Entnahme des Materials als solche. Das humane Material, bevorzugt Serum, Plasma oder Gewebe, wurde bevorzugt also bereits zuvor entnommen und der menschliche Körper, aus welchem das Material entnommen wurde, muss für die Durchführung des erfindungsgemäßen Verfahrens an sich nicht präsent sein. Somit beinhaltet Schritt (a) des erfindungsgemäßen Verfahrens die Bereitstellung des bereits entnommenen humanen Materials, wohingegen die Entnahme des humanen Materials an sich bevorzugt nicht Teil des erfindungsgemäßen Verfahrens ist.

Geeignete Verfahren zum Quantifizieren von FKBP1A, insbesondere zur Konzentrationsbestimmung von FKBP1A, sind einem Fachmann bekannt. In bevorzugten Ausführungsformen erfolgt Schritt (b) immunologisch durch FKBP1A-spezifischen ELISA (Serum, Plasma) oder immunhistologisch (Gewebe).

Ein weiterer nicht wörtlich beanspruchter Aspekt der Erfindung betrifft ein Verfahren zum Auffinden von Antikörpern, Proteinen oder Molekülen mit spezifischer Affinität zu FKBP1A, welche zur Behandlung von Erkrankungen, insbesondere Krebserkrankungen oder neurodegenerative Erkrankungen, geeignet sind, umfassend die Schritte
(a) Bereitstellen einer Bibliothek von Antikörpern, Antikörperfragmenten, Proteinen oder Proteinfragmenten;
(b) Selektieren von spezifischen FKBP1A-bindenden Antikörpern, Antikörperfragmenten, Proteinen oder Proteinfragmenten aus der Bibliothek;
(c) Isolieren und Sequenzieren der spezifischen FKBP1A-bindenden Antikörper, Antikörperfragmente, Proteine, Proteinfragmente oder deren codierender Gene aus Schritt (a); und
(d) Exprimieren der spezifischen FKBP1A-bindenden Antikörper, Antikörperfragmente, Proteine oder Proteinfragmente in geeigneten Wirtszellen.

Geeignete Methoden zum Bereitstellen von Antikörpern, Antikörperfragmenten, Proteinen oder Proteinfragmenten, zum Selektieren von spezifischen FKBP1A-bindenden Antikörpern, Antikörperfragmenten, Proteinen oder Proteinfragmenten, zum Isolieren und Sequenzieren sowie zum Exprimieren sind einem Fachmann bekannt.

In bevorzugten Ausführungsformen umfasst Schritt (b) die Selektion FKBP1A-bindender Phagen im Phagen-Display oder die Selektion durch Biopanning von Bibliotheken aus Antikörpern, Antikörperfragmenten, Proteinen und/oder Proteinfragmenten.

Ein weiterer nicht wörtlich beanspruchter Aspekt der Erfindung betrifft die Verwendung eines Antikörpers, Antikörperfragments, Proteins, Proteinfragments oder Moleküls mit spezifischer Affinität zu FKBP1A zur Herstellung eines Medikaments zur Therapie von Erkrankungen; insbesondere Krebserkrankungen; insbesondere FKBP1A-positiver Tumore; insbesondere des Triple-negativen Mammakarzinoms, des Her2-positiven Hormonrezeptor-negativen Mammakarzinoms oder des malignen Melanoms; insbesondere des Triple-negativen Mammakarzinoms, ganz insbesondere des TNBC-mesenchymal stem-like Subtyps.

Ein weiterer nicht wörtlich beanspruchter Aspekt der Erfindung betrifft die Verwendung eines Antikörpers, Antikörperfragments, Proteins, Proteinfragments oder Moleküls mit spezifischer Affinität zu FKBP1A zur Herstellung eines Medikaments umfassend ein Konjugat des Antikörpers, Proteins oder Moleküls mit einem Wirkstoff oder einem einen Wirkstoff enthaltenden speziellen Vesikel zum zielgerichteten Transport des Wirkstoffs.

Ein weiterer nicht wörtlich beanspruchter Aspekt der Erfindung betrifft ein solches Konjugat aus Antikörper, Protein oder Molekül mit spezifischer Affinität zu FKBP1A und Wirkstoff bzw. Wirkstoff enthaltenden Vesikels.

Ein weiterer nicht wörtlich beanspruchter Aspekt der Erfindung betrifft die Verwendung eines Antikörpers, Antikörperfragments, Proteins, Proteinfragments oder Moleküls mit spezifischer Affinität zu FKBP1A zur Herstellung eines Medikaments zur spezifischen Isolation und Charakterisierung von zirkulierenden Tumorzellen; insbesondere FKBP1A positiver Tumore; insbesondere des Triple-negativen Mammakarzinoms, des Her2-positiven Hormonrezeptor-negativen Mammakarzinoms oder des malignen Melanoms; insbesondere des Triple-negativen Mammakarzinoms, ganz insbesondere des TNBC-mesenchymal stem-like Subtyps; insbesondere zur Diagnose durch Liquid Biopsie.

Die nachfolgenden Beispiele dienen zur Erläuterung der Erfindung, sind jedoch nicht einschränkend auszulegen.

In den nachfolgenden Experimenten wurden folgende kommerzielle Zelllinien eingesetzt:

| | | Her2 | HR |
|---|---|---|---|
| MDA-MB-231 | Triple-negatives Mammakarzinom *mesenchymal stem-like* | negativ | negativ |
| MDA-MB-436 | Triple-negatives Mammakarzinom *mesenchymal stem-like* | negativ | negativ |
| MDA-MB-453 | Triple-negatives Mammakarzinom *androgen receptor positive* | (positiv) | negativ |
| MDA-MB-468 | Triple-negatives Mammakarzinom *basal-like* | negativ | negativ |
| MCF-7 | luminale Brustzell-Linie | negativ | positiv |
| MCF-10A | benigne Brustzell-Linie, d.h. nicht-maligne | positiv | positiv |
| SKBR3 | Mammakarzinom Her2-positiv | positiv | negativ |
| A375 | maligne Melanomzellen | | |

### Beispiel 1:

MDA-MB-231 (Mesenchymal stem-like), MDA-MB-436 (Mesenchymal stem-like), MDA-MB-468 (Basal-like), MCF-7: (luminal, HR-positiv) und MCF-10A (benigne Brustzell-Linie) (s. Abb. 1A) und MDA-MB-453, SKBR3 (Her2-positiv) und A375 (Melanoma) (s. Abb. 1B) wurden in einheitlichem Wachstumsmedium kultiviert (RPMI-1640-Medium, 10% FCS), mit PBS gewaschen, lysiert und mittels Western Blot analysiert.

Abbildung 1 zeigt eine Western-Blot Analyse der Proteinexpression von FKBP1A. Abbildung 1A zeigt das Ergebnis der Western-Blot Analyse für MDA-MB-231 und MDA-MB-436: Mesenchymal stem-like; MDA-MB-468: Basal-like; MCF-7: luminal, HR-positiv; MCF-10A: benigne Brustzell-Linie. Abbildung 1A zeigt, dass FKBP1A in Tumorzellen des Triple-negativen Mammakarzinoms in hohem Maße exprimiert wird, im Unterschied dazu in Tumorzellen von Hormonrezeptor-positiven Mammakarzinomen oder in nicht-malignen Zellen jedoch nicht nachweisbar ist. Abbildung 1A zeigt, dass FKBP1A stärker in "mesenchymal stem-like" triple-negativen Brustkrebszellen (MDA-MB-231 und MDA-MB-436) als in basal-like triple-negativen Brustkrebszellen (MDA-MB-468) exprimiert wird. Abbildung 1B zeigt das Ergebnis der Western-Blot Analyse für Her2-positive, HR-negative Brustkarzinom-Zellen. Abbildung 1B zeigt die hohe Expression von FKBP1A auch in Zellen des Her2-positiven, Hormonrezeptor-negativen Mammakarzinoms und des malignen Melanoms.

### Beispiel 2:

Eine Kultur von MDA-MB-231-Zellen wurde mit siRNA spezifisch gegen FKBP1A (S5202, Thermo Fisher) für drei Tage inkubiert, die Zellen mit PBS gewaschen und für weitere drei Tage ohne die Anwesenheit der siRNA inkubiert. Anschließend wurden die Zellen erneut mit PBS gewaschen, lysiert und mittels Western Blot die Expression von FKBP1A analysiert.

Abbildung 2 zeigt die Herabregulation der Proteinexpression von FKBP1A durch spezifische siRNA als Ergebnis einer Western-Blot Analyse der Proteinexpression von FKBP1A in MDA-MB-231-Zellen nach Inkubation mit FKBP1A-spezifischer siRNA (S5202, Thermo Fisher).

### Beispiel 3:

MDA-MB-231-Zellen wurden mit siRNA spezifisch gegen FKBPlA (S5202, Thermo Fisher) für drei Tage inkubiert, die Zellen mit PBS gewaschen und für weitere drei Tage ohne die Anwesenheit der siRNA inkubiert. Anschließend wurden die Zellen erneut mit PBS gewaschen, lysiert, die RNA extrahiert, cDNA synthetisiert und mittels qPCR die Expression unterschiedlicher (Tumor-)stammzell- und mesenchymaler Marker analysiert.

Abbildung 3 zeigt das Ergebnis einer Expressionsanalyse von (Tumor-)Stammzell- und mesenchymalen Markern nach FKBP1A knock-down. Die Expressionsanalyse erfolgte mittels quantitativer RT-PCR beschriebener Tumorstammzellmarker und des mesenchymalen Markers Vimentin relativ zu mock (=1) nach FKBP1A knock down mittels siRNA in MDA-MB-231-Zellen nach 6 Tagen. Abbildung 3A zeigt das Ergebnis normalisiert auf GAPDH. Abbildung 3B zeigt das Ergebnis normalisiert auf HRPT1.

### Beispiel 4:

Eine Kultur von MDA-MB-231-Zellen wurde mit Inhibitor I für sechs Tage inkubiert, wobei nach drei Tagen das Medium mit Anwesenheit des Inhibitors gewechselt wurde. Anschließend wurden die Zellen mit PBS gewaschen, lysiert und mittels Western Blot die Expression von FKBP1A analysiert.

Abbildung 4 zeigt die Herabregulation der Proteinexpression von FKBP1A durch Inhibitor (I) als Ergebnis einer Western-Blot Analyse der Proteinexpression von FKBP1A in MDA-MB-231-Zellen nach Inkubation mit 10 µM Inhibitor (I). Abbildung 4 zeigt, dass Wirkstoffe beim Triple-negativen Mammakarzinom eine Expressionsreduktion von FKBP1A induzieren können.

### Beispiel 5:

Eine Kultur von MDA-MB-231-Zellen wurde mit Inhibitor I für sechs Tage inkubiert, wobei nach drei Tagen das Medium mit Anwesenheit des Inhibitors gewechselt wurde. Anschließend wurden die Zellen erneut mit PBS gewaschen, lysiert, die RNA extrahiert, cDNA synthetisiert und mittels qPCR die Expression unterschiedlicher (Tumor-)stammzell- und mesenchymaler Marker sowie von PD-L1 analysiert.

Abbildung 5 zeigt das Ergebnis einer Expressionsanalyse von (Tumor-)Stammzell- und epithelialen Differenzierungsmarkern nach Expressionsreduktion von FKBP1A mittels Inhibitor (I). Die Expressionsanalyse erfolgte mittels quantitativer RT-PCR beschriebener (A) Tumorstammzell- und (B) Differenzierungsmarker sowie des (C) Immuncheckpoints PD-L1 nach 6h Inkubation von MDA-MB-231-Zellen mit Inhibitor (I). Abbildung 5 zeigt, dass Wirkstoffe beim Triple-negativen Mammakarzinom eine Expressionsreduktion von (Tumor-)Stammzellmarkern bewirken können.

## Patentansprüche

1. Ein Verfahren zum Auffinden von Inhibitoren der Peptidyl-prolyl cis-trans Isomerase FKBP1A, welche zur Behandlung des Triple-negativen Mammakarzinoms geeignet sind, umfassend die Schritte
(a) Bereitstellen eines Inhibitors
(i) der Expression von FKBP1A; und/oder
(ii) der enzymatischen Aktivität von FKBP1A; und/oder
(iii) der Interaktion(en) von FKBP1A mit Interaktionspartner(n);
(b) Inkubieren von Krebszellen einer Krebszelllinie mit dem Inhibitor von FKBP1A, und Ermitteln einer Eigenschaft der Krebszellen ausgewählt aus der Gruppe bestehend aus (A) zellulärem Differenzierungsstatus, (B) epithelialen Eigenschaften, (C) zellulärer Immunogenität, (D) Apoptoseinduzierung bzw. Apoptosefähigkeit und (E) zellulärem Stammzellcharakter;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b);
(d) Vergleichen der ermittelten Eigenschaft der Krebszellen gemäß Schritten (b) und (c).

2. Das Verfahren nach Anspruch 1, wobei Schritt (a) die Teilschritte umfasst
(a₁) Bereitstellen mehrerer Testsubstanzen;
(a₂) Durchmustern der Testsubstanzen hinsichtlich ihrer inhibierenden Wirkung auf
(i) die Expression von FKBP1A; und/oder
(ii) die enzymatische Aktivität von FKBP1A; und/oder
(iii) Interaktion(en) von FKBP1A mit Interaktionspartner(n);
(a₃) Auswählen mindestens einer durchmusterten Testsubstanz, deren inhibierende Wirkung stärker ist als die inhibierende Wirkung mindestens einer anderen durchmusterten Testsubstanz, und Bereitstellen dieser ausgewählten Testsubstanz als Inhibitor von FKBP1A.

3. Das Verfahren nach einem der vorstehenden Ansprüche, wobei der in Schritt (a) bereitgestellte Inhibitor
- die Interaktion(en) von FKBP1A mit Interaktionspartner(n) inhibiert und wobei mindestens ein Interaktionspartner ausgewählt ist aus der Gruppe bestehend aus Interaktionspartnern, welche eine Reduktion oder Arretierung (A) des zellulären Differenzierungsstatus', (B) der epithelialen Eigenschaften, (C) der zellulären Immunogenität (D) der Apoptoseinduzierung bzw. Apoptosefähigkeit und/oder (E) des zellulären Stammzellcharakters bewirken;
- die Interaktion(en) von FKBP1A mit Interaktionspartner(n) inhibiert und wobei mindestens ein Interaktionspartner ausgewählt ist aus der Gruppe bestehend aus interagierenden Proteinen, interagierenden Peptiden, und interagierenden Nukleinsäuren;
- die Expression von FKBP1A und nicht gleichzeitig (ii) die enzymatische Aktivität von FKB1A inhibiert; und/oder
- (ii) die enzymatische Aktivität von FKB1A und gleichzeitig nicht (i) die Expression von FKBP1A inhibiert.

4. Das Verfahren nach einem der vorstehenden Ansprüche, wobei
- in Schritt (b) die Eigenschaft ermittelt wird durch Quantifizierung von Differenzierungsmarkern, Epithelmarkern, (Tumor-)-Stammzellmarkern, immunmodulierenden Proteinen, immunvermittelnden Proteinen oder Kombinationen davon;
- Schritt (b) das Inkubieren einer Mischung von Krebszellen einer Krebszelllinie und Immunzellen einer Immunzelllinie mit dem Inhibitor von FKBP1A umfasst und wobei als Eigenschaft der Krebszellen deren zelluläre Immunogenität ermittelt wird durch Quantifizierung der Immunzellaktivierung und/oder durch Quantifizierung der immunzellvermittelten zytotoxischen Wirkung auf die Krebszellen; und wobei Schritt (c) das Inkubieren einer Mischung von Krebszellen der gleichen Krebszelllinie und Immunzellen der gleichen Immunzelllinie wie bei Schritt (b) in Abwesenheit des Inhibitors von FKBP1A und das Ermitteln der gleichen Eigenschaft der Krebszellen wie bei Schritt (b) unter gleichen Bedingungen wie Schritt (b) umfasst;
- die in Schritt (b) ermittelte Eigenschaft (A) zellulärer Differenzierungsstatus ist; bevorzugt wobei in Schritt (b) die Eigenschaft durch Quantifizierung von Mesenchymalmarkern ermittelt wird; bevorzugt durch Quantifizierung von Vimentin;
- die in Schritt (b) ermittelte Eigenschaft (C) zelluläre Immunogenität ist; bevorzugt wobei in Schritt (b) die Eigenschaft durch Quantifizierung von immunmodulierenden Proteinen ermittelt wird; bevorzugt durch Quantifizierung von PD-L1;
- die in Schritt (b) ermittelte Eigenschaft (E) zellulärer Stammzellcharakter ist; bevorzugt wobei in Schritt (b) die Eigenschaft durch Quantifizierung von (Tumor-)Stammzellmarkern ermittelt wird; bevorzugt durch Quantifizierung von CD44/CD24 und ALDH1; und/oder
- die in Schritt (b) ermittelte Eigenschaft die Quantifizierung der Expression von FKBP1A ist.

5. Das Verfahren nach einem der vorstehenden Ansprüche, umfassend die Schritte
(a) Bereitstellen eines Inhibitors (i) der Expression von FKBP1A;
bevorzugt wobei Schritt (a) die Teilschritte umfasst
(a₁) Bereitstellen mehrerer Testsubstanzen;
(a₂) Durchmustern der Testsubstanzen hinsichtlich ihrer inhibierenden Wirkung auf (i) die Expression von FKBP1A;
(a₃) Auswählen mindestens einer durchmusterten Testsubstanz, deren inhibierende Wirkung stärker ist als die inhibierende Wirkung mindestens einer anderen durchmusterten Testsubstanz, und Bereitstellen dieser ausgewählten Testsubstanz als Inhibitor von FKBP1A;
(b) Inkubieren von Krebszellen einer Krebszelllinie mit dem Inhibitor von FKBP1A, und Ermitteln einer Eigenschaft der Krebszellen;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b);
(d) Vergleichen der ermittelten Eigenschaft der Krebszellen gemäß Schritten (b) und (c).

6. Das Verfahren nach einem der vorstehenden Ansprüche, wobei die Krebszelllinie charakteristisch ist für das Triple-negative Mammakarzinom; bevorzugt eine Krebszelllinie des Triple-negativen Mammakarzinoms ist; bevorzugter eine Krebszelllinie des Triple-negativen Mammakarzinoms vom Subtyp mesenchymal stem-like ist.

7. Das Verfahren nach einem der vorstehenden Ansprüche, umfassend die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt den zellulären Differenzierungsstatus, bevorzugter durch Quantifizierung von Mesenchymalmarkern;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b);
(d) Vergleichen der ermittelten Eigenschaft der Krebszellen gemäß Schritten (b) und (c).

8. Das Verfahren nach einem der Ansprüche 1 bis 7, umfassend die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt zelluläre Immunogenität, bevorzugter durch Quantifizierung von immunmodulierenden Proteinen;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b);
(d) Vergleichen der ermittelten Eigenschaft der Krebszellen gemäß Schritten (b) und (c).

9. Das Verfahren nach einem der Ansprüche 1 bis 7, umfassend die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt zellulärer Stammzellcharakter, bevorzugter durch Quantifizierung von (Tumor-) Stammzellmarkern;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b);
(d) Vergleichen der ermittelten Eigenschaft der Krebszellen gemäß Schritten (b) und (c).

10. Das Verfahren nach einem der Ansprüche 1 bis 7, umfassend die Schritte
(a) Bereitstellen eines Inhibitors (i) der Expression von FKBP1A;
(b) Inkubieren von Krebszellen einer Krebszelllinie des Triple-negativen Mammakarzinoms, bevorzugt des Subtyps mesenchymal stem-like, bevorzugter der Krebszelllinie MDA-MB-231; mit dem Inhibitor von FKBP1A, und Ermitteln einer Eigenschaft der Krebszellen, bevorzugt die Quantifizierung der Expression von FKBP1A;
(c) Inkubieren von Krebszellen der gleichen Krebszelllinie wie bei Schritt (b) in Abwesenheit des Inhibitors von FKBP1A und Ermitteln der gleichen Eigenschaft der Krebszellen wie bei Schritt (b) unter gleichen Bedingungen wie Schritt (b);
(d) Vergleichen der ermittelten Eigenschaft der Krebszellen gemäß Schritten (b) und (c).

11. Das Verfahren nach einem der vorstehenden Ansprüche, wobei Schritt (a) die Teilschritte umfasst
(a₁) Bereitstellen mehrerer Testsubstanzen;
(a₂) Durchmustern der Testsubstanzen hinsichtlich ihrer inhibierenden Wirkung auf (i) die Expression von FKBP1A;
(a₃) Auswählen mindestens einer durchmusterten Testsubstanz, deren inhibierende Wirkung stärker ist als die inhibierende Wirkung mindestens einer anderen durchmusterten Testsubstanz, und Bereitstellen dieser ausgewählten Testsubstanz als Inhibitor von FKBP1A.

12. Das Verfahren nach einem der vorstehenden Ansprüche, wobei das Triple-negative Mammakarzinom dem mesenchymal stem-like Subtyp entspricht.

13. Eine FKBP1A-spezifische siRNA zur Anwendung bei der Behandlung des Triple-negativen Mammakarzinoms; bevorzugt wobei das Triple-negative Mammakarzinom dem mesenchymal stem-like Subtyp entspricht.

14. (A) Ein Inhibitor
(i) der Expression von FKBP1A; und/oder
(ii) der enzymatischen Aktivität von FKBP1A; und/oder
(iii) der Interaktion(en) von FKBP1A mit Interaktionspartner(n);
und/oder
(B) ein PROTAC (PROteolysis TArgeting Chimeras), welches spezifisch den zellulären Abbau von FKBP1A induziert, nämlich FKBP12 PROTAC RC32 (CAS No.: 2375555-66-9),
zur Anwendung bei der Behandlung des Triple-negativen Mammakarzinoms;
wobei der Inhibitor ein
- FK506; und/oder
- ein spezifischer Inhibitor von FKBP1A ohne immunsuppressive Wirkung zur synergistischen Immuntherapie, nämlich ElteN378, V-10367 (Vertex, USA), GPI-1046 (Guilford, USA), GPI-1485 (Guilford, USA), SLF, FK1706, FK-1012, AG5437 und/oder AG5507, ist.

15. Der Inhibitor zur Anwendung nach Anspruch 14 zur adjuvanten Behandlung des Triple-negativen Mammakarzinoms; bevorzugt zur (i) adjuvanten Behandlung bei einer Immuntherapie des Triple-negativen Mammakarzinoms und/oder zur (ii) Vermeidung von Metastasenbildung und/oder zur (iii) gezielten Therapie von Metastasen-induzierenden zirkulierenden Tumorzellen und/oder - zellverbänden (CTC und/oder CTC-Cluster), bevorzugt mit zusätzlicher Adressierung von PIN1.

## Claims

1. A method for finding inhibitors of peptidyl-prolyl cis-trans isomerase FKBP1A that are suitable for treating triple-negative breast cancer, comprising the steps of
(a) providing an inhibitor
(i) of the expression of FKBP1A; and/or
(ii) of the enzymatic activity of FKBP1A; and/or
(iii) of the interaction(s) of FKBP1A with interaction partner(s);
(b) incubating cancer cells of a cancer cell line with the inhibitor of FKBP1A, and determining a property of the cancer cells selected from the group consisting of (A) cellular differentiation status, (B) epithelial properties, (C) cellular immunogenicity, (D) apoptosis induction or apoptosis capacity, and (E) cellular stem cell character;
(c) incubating cancer cells of the same cancer cell line as in step (b) in the absence of the FKBP1A inhibitor and determining the same property of the cancer cells as in step (b) under the same conditions as step (b);
(d) comparing the determined property of the cancer cells according to steps (b) and (c).

2. The method according to claim 1, wherein step (a) comprises the sub-steps
(a₁) providing a plurality of test substances;
(a₂) screening the test substances for their inhibitory effect on
(i) the expression of FKBP1A; and/or
(ii) the enzymatic activity of FKBP1A; and/or
(iii) interaction(s) of FKBP1A with interaction partner(s);
(a₃) selecting at least one screened test substance whose inhibitory effect is stronger than the inhibitory effect of at least one other screened test substance, and providing this selected test substance as an inhibitor of FKBP1A.

3. The method according to any of the preceding claims, wherein the inhibitor provided in step (a)
- inhibits the interaction(s) of FKBP1A with interaction partner(s) and wherein at least one interaction partner is selected from the group consisting of interaction partners which reduce or arrest (A) the cellular differentiation status, (B) the epithelial properties, (C) the cellular immunogenicity, (D) the apoptosis induction or apoptosis capacity, and/or (E) the cellular stem cell character;
- inhibits the interaction(s) of FKBP1A with interaction partner(s), wherein at least one interaction partner is selected from the group consisting of interacting proteins, interacting peptides, and interacting nucleic acids;
- inhibits the expression of FKBP1A and not simultaneously (ii) the enzymatic activity of FKB1A; and/or
- (ii) inhibits the enzymatic activity of FKB1A and simultaneously does not inhibit (i) the expression of FKBP1A.

4. The method according to any of the preceding claims, wherein
- in step (b), the property is determined by quantifying differentiation markers, epithelial markers, (tumor) stem cell markers, immunomodulatory proteins, immune-mediating proteins, or combinations thereof;
- step (b) comprises incubating a mixture of cancer cells of a cancer cell line and immune cells of an immune cell line with the inhibitor of FKBP1A, and wherein the property of the cancer cells is determined as their cellular immunogenicity by quantifying immune cell activation and/or by quantifying the immune cell-mediated cytotoxic effect on the cancer cells; and wherein step (c) comprises incubating a mixture of cancer cells of the same cancer cell line and immune cells of the same immune cell line as in step (b) in the absence of the FKBP1A inhibitor and determining the same property of the cancer cells as in step (b) under the same conditions as step (b);
- the property determined in step (b) is (A) cellular differentiation status; preferably wherein in step (b) the property is determined by quantifying mesenchymal markers; preferably by quantifying vimentin;
- the property (C) determined in step (b) is cellular immunogenicity; preferably wherein in step (b) the property is determined by quantifying immunomodulatory proteins; preferably by quantifying PD-L1;
- the property (E) determined in step (b) is cellular stem cell character; preferably wherein in step (b) the property is determined by quantifying (tumor) stem cell markers; preferably by quantifying CD44/CD24 and ALDH1; and/or
- the property determined in step (b) is the quantification of the expression of FKBP1A.

5. The method according to any of the preceding claims, comprising the steps
(a) providing an inhibitor (i) of the expression of FKBP1A;
preferably wherein step (a) comprises the sub-steps
(a₁) providing a plurality of test substances;
(a₂) screening the test substances for their inhibitory effect on (i) the expression of FKBP1A;
(a₃) selecting at least one screened test substance whose inhibitory effect is stronger than the inhibitory effect of at least one other screened test substance, and providing this selected test substance as an inhibitor of FKBP1A;
(b) incubating cancer cells of a cancer cell line with the FKBP1A inhibitor and determining a property of the cancer cells;
(c) incubating cancer cells of the same cancer cell line as in step (b) in the absence of the FKBP1A inhibitor and determining the same property of the cancer cells as in step (b) under the same conditions as step (b);
(d) comparing the determined property of the cancer cells according to steps (b) and (c).

6. The method according to any of the preceding claims, wherein the cancer cell line is characteristic of triple-negative breast cancer; preferably is a cancer cell line of triple-negative breast cancer; more preferably is a cancer cell line of triple-negative breast cancer of the mesenchymal stem-like subtype.

7. The method according to any of the preceding claims, comprising the steps
(a) providing an inhibitor (i) of FKBP1A expression;
(b) incubating cancer cells of a cancer cell line of triple-negative breast cancer, preferably of the mesenchymal stem-like subtype, more preferably of the MDA-MB-231 cancer cell line; with the inhibitor of FKBP1A, and determining a property of the cancer cells, preferably the cellular differentiation status, more preferably by quantifying mesenchymal markers;
(c) incubating cancer cells of the same cancer cell line as in step (b) in the absence of the FKBP1A inhibitor and determining the same property of the cancer cells as in step (b) under the same conditions as step (b);
(d) comparing the determined property of the cancer cells according to steps (b) and (c).

8. The method according to any one of claims 1 to 7, comprising the steps of
(a) providing an inhibitor (i) of FKBP1A expression;
(b) incubating cancer cells of a triple-negative breast cancer cell line, preferably of the mesenchymal stem-like subtype, more preferably of the MDA-MB-231 cancer cell line, with the FKBP1A inhibitor, and determining a property of the cancer cells, preferably cellular immunogenicity, more preferably by quantifying immunomodulatory proteins;
(c) incubating cancer cells of the same cancer cell line as in step (b) in the absence of the FKBP1A inhibitor and determining the same property of the cancer cells as in step (b) under the same conditions as step (b);
(d) comparing the determined property of the cancer cells according to steps (b) and (c).

9. The method according to any one of claims 1 to 7, comprising the steps of
(a) providing an inhibitor (i) of FKBP1A expression;
(b) incubating cancer cells of a triple-negative breast cancer cell line, preferably of the mesenchymal stem-like subtype, more preferably of the MDA-MB-231 cancer cell line, with the FKBP1A inhibitor, and determining a property of the cancer cells, preferably cellular stem cell character, more preferably by quantifying (tumor) stem cell markers;
(c) incubating cancer cells of the same cancer cell line as in step (b) in the absence of the FKBP1A inhibitor and determining the same property of the cancer cells as in step (b) under the same conditions as step (b);
(d) comparing the determined property of the cancer cells according to steps (b) and (c).

10. The method according to any one of claims 1 to 7, comprising the steps of
(a) providing an inhibitor (i) of FKBP1A expression;
(b) incubating cancer cells of a triple-negative breast cancer cell line, preferably of the mesenchymal stem-like subtype, more preferably of the MDA-MB-231 cancer cell line, with the FKBP1A inhibitor, and determining a property of the cancer cells, preferably quantifying the expression of FKBP1A;
(c) incubating cancer cells of the same cancer cell line as in step (b) in the absence of the FKBP1A inhibitor and determining the same property of the cancer cells as in step (b) under the same conditions as step (b);
(d) comparing the determined property of the cancer cells according to steps (b) and (c).

11. The method according to any of the preceding claims, wherein step (a) comprises the sub-steps
(a₁) providing a plurality of test substances;
(a₂) screening the test substances for their inhibitory effect on (i) the expression of FKBP1A;
(a₃) selecting at least one screened test substance whose inhibitory effect is stronger than the inhibitory effect of at least one other screened test substance, and providing this selected test substance as an inhibitor of FKBP1A.

12. The method according to any of the preceding claims, wherein the triple-negative breast cancer corresponds to the mesenchymal stem-like subtype.

13. An FKBP1A-specific siRNA for use in the treatment of triple-negative breast cancer; preferably wherein the triple-negative breast cancer corresponds to the mesenchymal stem-like subtype.

14. (A) An inhibitor
(i) of the expression of FKBP1A; and/or
(ii) of the enzymatic activity of FKBP1A; and/or
(iii) of the interaction(s) of FKBP1A with interaction partner(s);
and/or
(B) a PROTAC (PROteolysis TArgeting Chimeras) that specifically induces the cellular degradation of FKBP1A, namely FKBP12 PROTAC RC32 (CAS No.: 2375555-66-9),
for use in the treatment of triple-negative breast cancer;
wherein the inhibitor is a
- FK506; and/or
- a specific inhibitor of FKBP1A without immunosuppressive effect for synergistic immunotherapy, namely ElteN378, V-10367 (Vertex, USA), GPI-1046 (Guilford, USA), GPI-1485 (Guilford, USA), SLF, FK1706, FK-1012, AG5437 and/or AG5507.

15. The inhibitor for use according to claim 14 for the adjuvant treatment of triple-negative breast cancer; preferably for (i) adjuvant treatment in immunotherapy of triple-negative breast cancer and/or for (ii) preventing metastasis formation and/or for (iii) targeted therapy of metastasis-inducing circulating tumor cells and/or cell clusters (CTC and/or CTC clusters), preferably with additional targeting of PIN1.

## Revendications

1. Procédé de découverte d'inhibiteurs de la peptidyl-prolyl cis-trans isomérase FKBP1A, qui sont adaptés au traitement du cancer du sein triple négatif, comprenant les étapes suivantes
(a) fournir un inhibiteur
(i) l'expression de FKBP1A; et/ou
(ii) de l'activité enzymatique de FKBP1A; et/ou
(iii) l'interaction ou les interactions de FKBP1A avec un ou plusieurs partenaires d'interaction;
(b) incuber des cellules cancéreuses d'une lignée cellulaire cancéreuse avec l'inhibiteur de FKBP1A, et la détermination d'une propriété des cellules cancéreuses choisie dans le groupe constitué par (A) le statut de différenciation cellulaire, (B) les propriétés épithéliales, (C) l'immunogénicité cellulaire, (D) l'induction de l'apoptose ou la capacité d'apoptose et (E) le caractère de cellule souche cellulaire;
(c) incuber des cellules cancéreuses de la même lignée cellulaire cancéreuse que dans l'étape (b) en l'absence de l'inhibiteur de FKBP1A et déterminer la même propriété des cellules cancéreuses que dans l'étape (b) dans les mêmes conditions que l'étape (b);
(d) comparer la propriété déterminée des cellules cancéreuses selon les étapes (b) et (c).

2. Procédé selon la revendication 1, dans lequel l'étape (a) comprend les sous-étapes suivantes
(a₁) fournir plusieurs substances d'essai;
(a₂) d'examiner les substances d'essai en ce qui concerne leur effet inhibiteur sur
(i) l'expression de FKBP1A; et/ou
(ii) l'activité enzymatique de FKBP1A; et/ou
(iii) l'interaction ou les interactions de FKBP1A avec un ou plusieurs partenaires d'interaction;
(a₃) sélectionner au moins une substance testée dont l'effet inhibiteur est plus fort que l'effet inhibiteur d'au moins une autre substance testée, et fournir cette substance testée sélectionnée comme inhibiteur de FKBP1A.

3. Procédé selon l'une des revendications précédentes, dans lequel l'inhibiteur fourni à l'étape (a)
- inhibe la ou les interactions de FKBP1A avec un ou plusieurs partenaires d'interaction, et dans lequel au moins un partenaire d'interaction est sélectionné dans le groupe constitué des partenaires d'interaction qui provoquent une réduction ou un blocage (A) du statut de différenciation cellulaire, (B) des propriétés épithéliales, (C) de l'immunogénicité cellulaire, (D) de l'induction de l'apoptose ou de la capacité d'apoptose et/ou (E) du caractère de cellule souche cellulaire;
- inhibe la ou les interactions de FKBP1A avec le ou les partenaires d'interaction, au moins un partenaire d'interaction étant choisi dans le groupe constitué par les protéines interactives, les peptides interactifs et les acides nucléiques interactifs;
- inhibe l'expression de FKBP1A et non simultanément (ii) l'activité enzymatique de FKB1A; et/ou
- (ii) l'activité enzymatique de FKB1A et simultanément non (i) l'expression de FKBP1A.

4. Procédé selon l'une des revendications précédentes, dans lequel
- à l'étape (b), la propriété est déterminée par quantification de marqueurs de différenciation, de marqueurs épithéliaux, de marqueurs de cellules souches (tumorales), de protéines immunomodulatrices, de protéines immunitaires ou de combinaisons de ceux-ci;
- l'étape (b) comprend l'incubation d'un mélange de cellules cancéreuses d'une lignée cellulaire cancéreuse et de cellules immunitaires d'une lignée cellulaire immunitaire avec l'inhibiteur de FKBP1A, et dans lequel la propriété des cellules cancéreuses est déterminée par quantification de l'immunogénicité cellulaire et/ou par quantification de l'effet cytotoxique à médiation immunitaire sur les cellules cancéreuses; et dans lequel l'étape (c) comprend l'incubation d'un mélange de cellules cancéreuses de la même lignée cellulaire cancéreuse et de cellules immunitaires de la même lignée cellulaire immunitaire que dans l'étape (b) en l'absence de l'inhibiteur de FKBP1A et la détermination de la même propriété des cellules cancéreuses que dans l'étape (b) dans les mêmes conditions que l'étape (b);
- la propriété déterminée à l'étape (b) (A) est le statut de différenciation cellulaire; de préférence, à l'étape (b), la propriété est déterminée par quantification des marqueurs mésenchymateux; de préférence par quantification de la vimentine;
- la propriété (C) déterminée à l'étape (b) est l'immunogénicité cellulaire; de préférence, à l'étape (b), la propriété est déterminée par quantification des protéines immunomodulatrices; de préférence par quantification du PD-L1;
- la propriété (E) du caractère des cellules souches déterminée à l'étape (b); de préférence, à l'étape (b), la propriété est déterminée par quantification des marqueurs des cellules souches (tumorales); de préférence par quantification de CD44/CD24 et ALDH1; et/ou
- la propriété déterminée à l'étape (b) est la quantification de l'expression de FKBP1A.

5. Procédé selon l'une des revendications précédentes, comprenant les étapes
(a) fournir un inhibiteur (i) de l'expression de FKBP1A;
de préférence l'étape (a) comprenant les sous-étapes
(a₁) fournir plusieurs substances d'essai;
(a₂) le criblage des substances d'essai en ce qui concerne leur effet inhibiteur sur (i) l'expression de FKBP1A;
(a₃) Sélection d'au moins une substance testée dont l'effet inhibiteur est plus fort que l'effet inhibiteur d'au moins une autre substance testée, et fourniture de cette substance testée sélectionnée comme inhibiteur de FKBP1A;
(b) Incuber des cellules cancéreuses d'une lignée cellulaire cancéreuse avec l'inhibiteur de FKBP1A et déterminer une propriété des cellules cancéreuses;
(c) Incuber des cellules cancéreuses de la même lignée cellulaire cancéreuse que dans l'étape (b) en l'absence de l'inhibiteur de FKBP1A et déterminer la même propriété des cellules cancéreuses que dans l'étape (b) dans les mêmes conditions que l'étape (b);
(d) comparer la propriété déterminée des cellules cancéreuses selon les étapes (b) et (c).

6. Procédé selon l'une des revendications précédentes, dans lequel la lignée cellulaire cancéreuse est caractéristique du cancer du sein triple négatif; de préférence, est une lignée cellulaire cancéreuse du cancer du sein triple négatif; de préférence, est une lignée cellulaire cancéreuse du cancer du sein triple négatif du sous-type mésenchymateux de type souche.

7. Procédé selon l'une des revendications précédentes, comprenant les étapes suivantes
(a) fournir un inhibiteur (i) de l'expression de FKBP1A;
(b) l'incubation de cellules cancéreuses d'une lignée cellulaire cancéreuse du cancer du sein triple négatif, de préférence du sous-type mésenchymateux de type souche, de préférence de la lignée cellulaire cancéreuse MDA-MB-231; avec l'inhibiteur de FKBP1A, et la détermination d'une propriété des cellules cancéreuses, de préférence le statut de différenciation cellulaire, de préférence par quantification des marqueurs mésenchymateux;
(c) incuber des cellules cancéreuses de la même lignée cellulaire cancéreuse que dans l'étape (b) en l'absence de l'inhibiteur de FKBP1A et déterminer la même propriété des cellules cancéreuses que dans l'étape (b) dans les mêmes conditions que l'étape (b);
(d) comparer la propriété déterminée des cellules cancéreuses selon les étapes (b) et (c).

8. Procédé selon l'une des revendications 1 à 7, comprenant les étapes suivantes
(a) fournir un inhibiteur (i) de l'expression de FKBP1A;
(b) l'incubation de cellules cancéreuses d'une lignée cellulaire cancéreuse du cancer du sein triple négatif, de préférence du sous-type mésenchymateux de type souche, de préférence de la lignée cellulaire cancéreuse MDA-MB-231; avec l'inhibiteur de FKBP1A, et la détermination d'une propriété des cellules cancéreuses, de préférence l'immunogénicité cellulaire, de préférence par quantification des protéines immunomodulatrices;
(c) Incuber des cellules cancéreuses de la même lignée cellulaire cancéreuse que dans l'étape (b) en l'absence de l'inhibiteur de FKBP1A et déterminer la même propriété des cellules cancéreuses que dans l'étape (b) dans les mêmes conditions que l'étape (b);
(d) comparer la propriété déterminée des cellules cancéreuses selon les étapes (b) et (c).

9. Procédé selon l'une des revendications 1 à 7, comprenant les étapes suivantes
(a) fournir un inhibiteur (i) de l'expression de FKBP1A;
(b) l'incubation de cellules cancéreuses d'une lignée cellulaire cancéreuse du cancer du sein triple négatif, de préférence du sous-type mésenchymateux de type souche, de préférence de la lignée cellulaire cancéreuse MDA-MB-231; avec l'inhibiteur de FKBP1A, et la détermination d'une propriété des cellules cancéreuses, de préférence le caractère de cellule souche cellulaire, de préférence par quantification des marqueurs de cellules souches (tumorales);
(c) Incuber des cellules cancéreuses de la même lignée cellulaire cancéreuse que dans l'étape (b) en l'absence de l'inhibiteur de FKBP1A et déterminer la même propriété des cellules cancéreuses que dans l'étape (b) dans les mêmes conditions que l'étape (b);
(d) comparer la propriété déterminée des cellules cancéreuses selon les étapes (b) et (c).

10. Procédé selon l'une des revendications 1 à 7, comprenant les étapes suivantes
(a) fournir un inhibiteur (i) de l'expression de FKBP1A;
(b) l'incubation de cellules cancéreuses d'une lignée cellulaire cancéreuse du cancer du sein triple négatif, de préférence du sous-type mésenchymateux de type souche, de préférence de la lignée cellulaire cancéreuse MDA-MB-231; avec l'inhibiteur de FKBP1A, et la détermination d'une propriété des cellules cancéreuses, de préférence la quantification de l'expression de FKBP1A;
(c) incuber les cellules cancéreuses de la même lignée cellulaire cancéreuse que dans l'étape (b) en l'absence de l'inhibiteur de FKBP1A et déterminer la même propriété des cellules cancéreuses que dans l'étape (b) dans les mêmes conditions que l'étape (b);
(d) comparaison de la propriété déterminée des cellules cancéreuses selon les étapes (b) et (c).

11. Procédé selon l'une des revendications précédentes, dans lequel l'étape (a) comprend les sous-étapes suivantes
(a₁) fournir plusieurs substances d'essai;
(a₂) l'examen des substances d'essai en ce qui concerne leur effet inhibiteur sur (i) l'expression de FKBP1A;
(a₃) sélectionner au moins une substance testée dont l'effet inhibiteur est plus fort que l'effet inhibiteur d'au moins une autre substance testée, et fournir cette substance testée sélectionnée comme inhibiteur de FKBP1A.

12. Procédé selon l'une des revendications précédentes, dans lequel le cancer du sein triple négatif correspond au sous-type mésenchymateux de type souche.

13. Un ARNsi spécifique de FKBP1A destiné à être utilisé dans le traitement du cancer du sein triple négatif; de préférence, le cancer du sein triple négatif correspondant au sous-type mésenchymateux de type souche.

14. (A) Un inhibiteur
(i) de l'expression de FKBP1A; et/ou
(ii) de l'activité enzymatique de FKBP1A; et/ou
(iii) l'interaction ou les interactions de FKBP1A avec un ou plusieurs partenaires d'interaction;
et/ou
(B) un PROTAC (PROteolysis TArgeting Chimeras) qui induit spécifiquement la dégradation cellulaire de FKBP1A, à savoir FKBP12 PROTAC RC32 (n° CAS : 2375555-66-9),
destiné à être utilisé dans le traitement du cancer du sein triple négatif;
dans lequel l'inhibiteur est un
- FK506; et/ou
- un inhibiteur spécifique de FKBP1A sans effet immunosuppresseur pour une immunothérapie synergique, à savoir ElteN378, V-10367 (Vertex, États-Unis), GPI-1046 (Guilford, États-Unis), GPI-1485 (Guilford, États-Unis), SLF, FK1706, FK-1012, AG5437 et/ou AG5507.

15. L'inhibiteur destiné à être utilisé selon la revendication 14 pour le traitement adjuvant du cancer du sein triple négatif; de préférence pour (i) le traitement adjuvant dans le cadre d'une immunothérapie du cancer du sein triple négatif et/ou pour (ii) la prévention de la formation de métastases et/ou pour (iii) thérapie ciblée des cellules tumorales circulantes et/ou des groupes de cellules tumorales (CTC et/ou CTC-Cluster) induisant des métastases, de préférence avec un ciblage supplémentaire de PIN1.
